(19) Europäisches Patentamt
European Patent Office
Office européen des brevets

(11) **EP 1 753 810 B1**

(12) **EUROPEAN PATENT SPECIFICATION**

(45) Date of publication and mention
of the grant of the patent:
**14.08.2013 Bulletin 2013/33**

(51) Int Cl.:
*C08L 5/08* (2006.01)          *C08B 37/08* (2006.01)
*A61K 9/16* (2006.01)          *A61K 8/73* (2006.01)
*A61Q 19/00* (2006.01)

(21) Application number: **05744576.9**

(86) International application number:
**PCT/DK2005/000350**

(22) Date of filing: **26.05.2005**

(87) International publication number:
**WO 2005/116131 (08.12.2005 Gazette 2005/49)**

(54) **A DRIED AND AGGLOMERATED HYALURONIC ACID PRODUCT**

GETROCKNETES UND AGGLOMERIERTES HYALURONSÄUREPRODUKT

PRODUIT D'ACIDE HYALURONIQUE SECHE ET AGGLOMERE

(84) Designated Contracting States:
**AT BE BG CH CY CZ DE DK EE ES FI FR GB GR
HU IE IS IT LI LT LU MC NL PL PT RO SE SI SK TR**

(30) Priority: **27.05.2004 DK 200400833
25.06.2004 DK 200401003
15.02.2005 DK 200500223**

(43) Date of publication of application:
**21.02.2007 Bulletin 2007/08**

(73) Proprietor: **Novozymes Biopharma DK A/S
2880 Bagsvaerd (DK)**

(72) Inventors:
• **BACH, Poul
DK-3460 Birkerød (DK)**

• **THWAITES, Eric
DK-2300 Copenhagen (DK)**

(74) Representative: **Kofoed, Gertrud Sonne et al
Novozymes A/S
Patents
Krogshoejvej 36
2880 Bagsvaerd (DK)**

(56) References cited:
**WO-A-02/09787          WO-A-03/080827
GB-A- 2 218 429          US-A- 3 687 640
US-A- 4 754 027          US-A- 5 095 037
US-A- 5 171 562**

**Description**

**FIELD OF INVENTION**

The present invention relates to a dried and agglomerated product comprising hyaluronic acid or a salt thereof, as well as to various compositions and articles comprising the product or compositions of the invention, methods of producing the product of the invention, and uses thereof.

**BACKGROUND**

The most abundant heteropolysaccharides of the body are the glycosaminoglycans. Glycosaminoglycans are unbranched carbohydrate polymers, consisting of repeating disaccharide units (only keratan sulphate is branched in the core region of the carbohydrate). The disaccharide units generally comprise, as a first saccharide unit, one of two modified sugars - N-acetylgalactosamine (GalNAc) or N-acetylglucosamine (GlcNAc). The second unit is usually an uronic acid, such as glucuronic acid (GlcUA) or iduronate.

Glycosaminoglycans are negatively charged molecules, and have an extended conformation that imparts high viscosity when in solution. Glycosaminoglycans are located primarily on the surface of cells or in the extracellular matrix. Glycosaminoglycans also have low compressibility in solution and, as a result, are ideal as a physiological lubricating fluid, e.g., joints. The rigidity of glycosaminoglycans provides structural integrity to cells and provides passageways between cells, allowing for cell migration. The glycosaminoglycans of highest physiological importance are hyaluronan, chondroitin sulfate, heparin, heparan sulfate, dermatan sulfate, and keratan sulfate. Most glycosaminoglycans bind covalently to a proteoglycan core protein through specific oligosaccharide structures. Hyaluronan forms large aggregates with certain proteoglycans, but is an exception as free carbohydrate chains form non-covalent complexes with proteoglycans.

Numerous roles of hyaluronan in the body have been identified (see, Laurent T. C. and Fraser J. R. E., 1992, FASEB J. 6: 2397-2404; and Toole B.P., 1991, "Proteoglycans and hyaluronan in morphogenesis and differentiation." In: Cell Biology of the Extracellular Matrix, pp. 305-341, Hay E. D., ed., Plenum, New York). Hyaluronan is present in hyaline cartilage, synovial joint fluid, and skin tissue, both dermis and epidermis. Hyaluronan is also suspected of having a role in numerous physiological functions, such as adhesion, development, cell motility, cancer, angiogenesis, and wound healing. Due to the unique physical and biological properties of hyaluronan, it is employed in eye and joint surgery and is being evaluated in other medical procedures.

The terms "hyaluronan" or "hyaluronic acid" are used in literature to mean acidic polysaccharides with different molecular weights constituted by residues of D-glucuronic and N-acetyl-D-glucosamine acids, which occur naturally in cell surfaces, in the basic extracellular substances of the connective tissue of vertebrates, in the synovial fluid of the joints, in the endobulbar fluid of the eye, in human umbilical cord tissue and in cocks' combs.

The term "hyaluronic acid" is in fact usually used as meaning a whole series of polysaccharides with alternating residues of D-glucuronic and N-acetyl-D-glucosamine acids with varying molecular weights or even the degraded fractions of the same, and it would therefore seem more correct to use the plural term of "hyaluronic acids". The singular term will, however, be used all the same in this description; in addition, the abbreviation "HA" will frequently be used in place of this collective term.

HA plays an important role in the biological organism, as a mechanical support for the cells of many tissues, such as the skin, tendons, muscles and cartilage, it is a main component of the intercellular matrix. HA also plays other important parts in the biological processes, such as the moistening of tissues, and lubrication.

HA may be extracted from the above mentioned natural tissues, although today it is preferred to prepare it by microbiological methods to minimize the potential risk of transferring infectious agents, and to increase product uniformity, quality and availability.

HA and its various molecular size fractions and the respective salts thereof have been used as medicaments, especially in treatment of arthropathies, as an auxiliary and/or substitute agent for natural organs and tissues, especially in ophtalmology and cosmetic surgery, and as agents in cosmetic preparations. Products of hyaluronan have also been developed for use in orthopaedics, rheumatology, and dermatology.

HA may also be used as an additive for various polymeric materials used for sanitary and surgical articles, such as polyurethanes, polyesters etc. with the effect of rendering these materials biocompatible.

Water soluble gel forming materials comprising hyaluronic acid or salts thereof are well-known and used widely in the health care sector, e.g., in ophtalmology, in the treatment of osteoarthritis, as well as in the pharmaceutical and cosmetics industries. These materials are ideally sold, transported, and stored in a dried form. However, dried products comprising hyaluronic acid or salts thereof have been notoriously slowly dissolving.
The time it takes for a dried HA-product to be completely dissolved is an important parameter, especially in the health care industry, therefor it is of considerable interest to minimize the total dissolution time, i.e., the time it takes to disperse and solubilize a dried HA product. This invention is useful for manufacturing fast dissolving products of hyaluronic acid or salts thereof, and similar water soluble gel forming materials.

GB 2 218 429 discloses a process for producing finely powdered hyaluronic acid or sodium hyaluronate comprising suspending hyaluronic acid or sodium hyaluronate in an organic solvent, grinding the suspension with a wet type grinder, removing the organic solvent, and drying the ground hyaluronic acid or sodium hyaluronate.

US 4,754,027 discloses a process for preparing an agglomerated guar-gum particulate product having a particle size in the range of from 0.5 to 3 millimeters.

WO 02/09787 discloses a process for fabrication of hyaluronic acid microspheres wherein a dispersion or colloidal solution of hyaluronic acid is sprayed to physically form microspheres of a desired size.

## SUMMARY OF THE INVENTION

A problem to be solved by the present invention is how to provide a highly water-soluble dried HA product.

The present invention shows that agglomeration of a dried HA product greatly improves the solubility of the product, thus achieving very fast dissolution in an aqueous solvent, which preferably is water or saline water.

Accordingly, in a first aspect the invention relates to a product comprising hyaluronic acid or a salt thereof, which product is dried and agglomerated, preferably dried and agglomerated as disclosed herein.

In a second aspect, the invention relates to a composition comprising a product as defined in the first aspect, and an active ingredient, preferably the active ingredient is a pharmacologically active agent.

A third aspect of the invention relates to a pharmaceutical composition comprising an effective amount of a product as defined in the first aspect, together with a pharmaceutically acceptable carrier, excipient or diluent.

A fourth aspect relates to a pharmaceutical composition comprising an effective amount of a product as defined in the first aspect as a vehicle, together with a pharmacologically active agent.

A fifth asepct relates to a cosmetic article comprising as an active ingredient an effective amount of a product as defined in the first aspect or a composition as defined in any of the second, third, or fourth aspects.

In a sixth aspect, the invention relates to a sanitary, medical or surgical article comprising a product as defined in the first aspect or a composition as defined in any of the second, third, or fourth aspects, preferably the article is a diaper, a sanitary towel, a surgical sponge, a wound healing sponge, or a part comprised in a band aid or other wound dressing material.

An important aspect relatest to a medicament capsule or microcapsule comprising a product as defined in the first aspect or a composition as defined in any of the second, third, or fourth aspects.

Another important aspect of the invention relates to a method of producing a dried and agglomerated product comprising hyaluronic acid or a salt thereof, the method comprising the steps of:

    a) drying a product comprising hyaluronic acid or a salt thereof; and
    b) agglomerating the dried product.

Final aspects of the invention relate to methods of performing procedures in ophtalmology, in the treatment of osteoarthritis or cancer, hair loss or baldness, of treating a wound, of performing dermal or transdermal administration of a pharmacologically active agent, or dermal administration of a cosmetic, the improvement which comprises the use of a product as defined in the first aspect, or a composition as defined in any of the second, third, or fourth aspects.

A number of aspects relate to uses of a product as defined in any of claims 1-12 or a composition as defined in any of claims 13 - 16, for the manufacture of a medicament for the treatment of osteoarthritis, cancer, the manufacture of a medicament for an ophtalmological treatment, the manufacture of a medicament for the treatment of a wound, the manufacture of a medicament for angiogenesis, the manufacture of a medicament for the treatment of hair loss or baldness, or the manufacture of a moisturizer.

## FIGURES

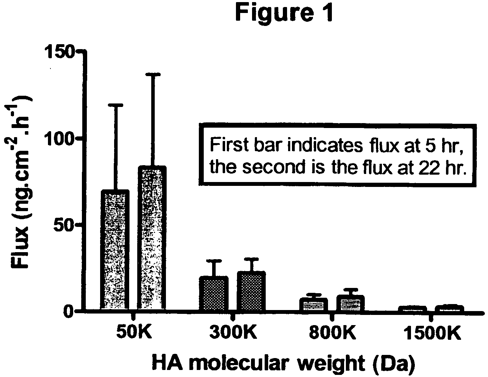

Figure 1: Skin permeation of four different average molecular weight fractions of radiolabeled hyaluronic acid was investigated as described in example 7. The results are shown in table 5, and visualized in figure 1. They indicate that there is an apparent dependence of percutaneous transport (flux) on HA molecular weight, with a better permeation apparent for the lower molecular weight species. There was no significant difference between the apparent fluxes measured at 5 and 22 hours for any of the HA size fractions studied.

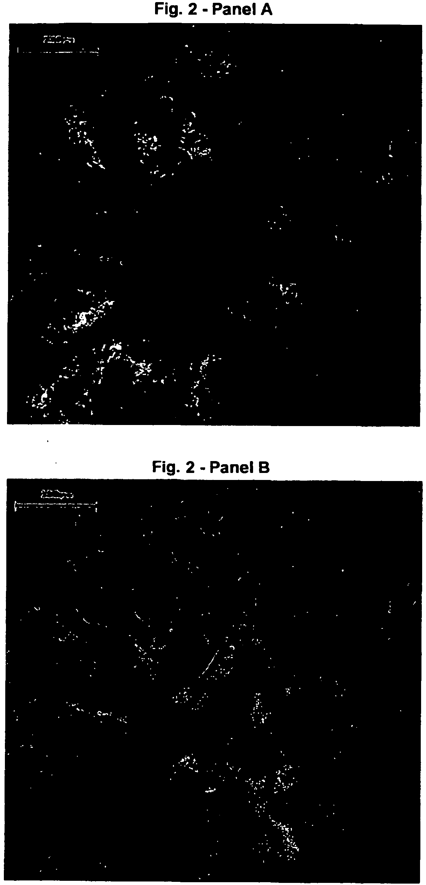

Figure 2: Skin uptake and distribution of two different average molecular weight fractions of flourescently labelled hyaluronic acid was investigated in example 8, using laser scanning confocal microscopy. The average molecular weights of the HA fractions were 300,000 and 50,000 Da. Flourescently labelled HA of 50,000 Da (F-HA 0.05) was only sparsely and superficially visible on the surface of the stratum corneum, but the labelled HA fraction was clearly visible around and/or in hair follicles. Figure 2, panel A, shows skin surface (stratum corneum), hair follicle and hair shaft; panel B clearly shows that green flourescence from HA is primarily visible around and/or in the follicle.

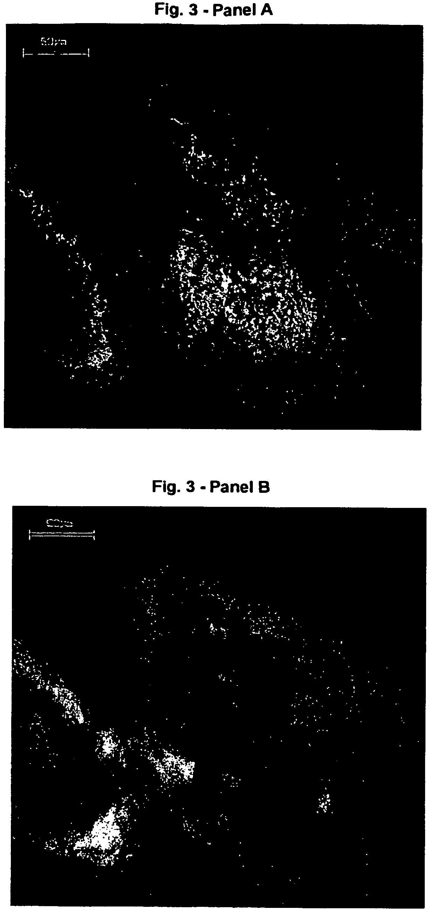

Figure 3: The sparse flourescence on the surface of the stratum corneum seen in figure 2 was confirmed in another skin sample, which did not have a follicle present. Figure 3, panel A, shows a skin surface; Figure 3 panel B shows, that only limited flourescence from labelled HA is visible on that surface.

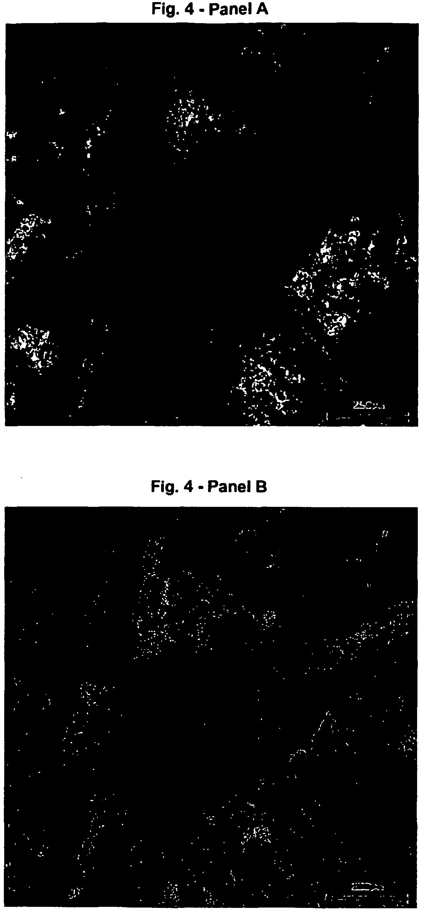

Figure 4: A similar observation was made with the flourescently labelled HA of 300,000 Da (F-HA 0.30), which also was clearly visible around the hair follicles. Figure 4, panel A, shows a skin surface (stratum corneum), hair follicle and hair shaft; panel B shows the green flourescence from labelled HA around the follicle.

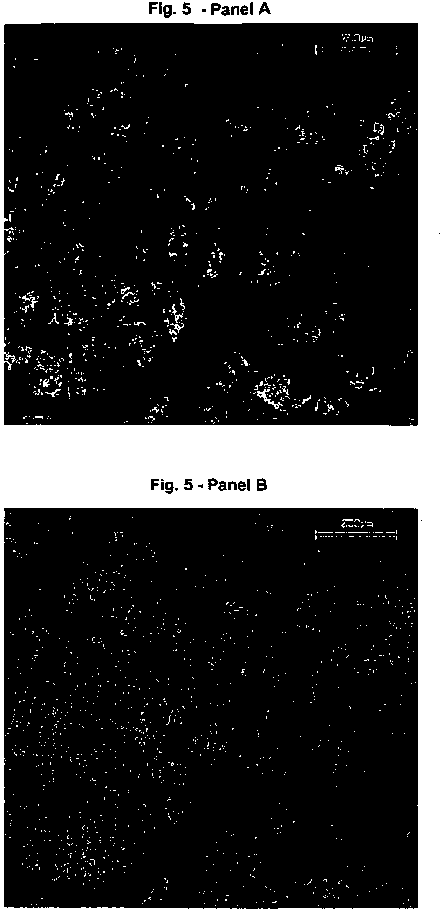

Figure 5: However, while the labelled HA fraction of 300,000 Da was also only sparsely and superficially visible on the skin surface, it clearly showed a preferential accumulation between the keratinocytes on the skin surface. Figure 5, panel A, shows a skin surface; panel B shows that while limited flourescence from labelled HA is visible on that surface, it is accumulated between the keratinocytes.

## DEFINITIONS

Nucleic Acid Constructs

"Nucleic acid construct" is defined herein as a nucleic acid molecule, either single-or double-stranded, which is isolated from a naturally occurring gene or which has been modified to contain segments of nucleic acid which are combined and juxtaposed in a manner which would not otherwise exist in nature. The term nucleic acid construct may be synonymous with the term expression cassette when the nucleic acid construct contains all the control sequences required for expression of a coding sequence. The term "coding sequence" is defined herein as a sequence which is transcribed into mRNA and translated into an enzyme of interest when placed under the control of the below mentioned control sequences. The boundaries of the coding sequence are generally determined by a ribosome binding site located just upstream of the open reading frame at the 5' end of the mRNA and a transcription terminator sequence located just downstream of the open reading frame at the 3' end of the mRNA. A coding sequence can include, but is not limited to, DNA, cDNA, and recombinant nucleic acid sequences.

The techniques used to isolate or clone a nucleic acid sequence encoding a polypeptide are well known in the art and include, for example, isolation from genomic DNA, preparation from cDNA, or a combination thereof. The cloning of the nucleic acid sequences from such genomic DNA can be effected, e.g., by using antibody screening of expression libraries to detect cloned DNA fragments with shared structural features or the well known polymerase chain reaction (PCR). See, for example, Innis et al., 1990, PCR Protocols: A Guide to Methods and Application, Academic Press, New York. Other nucleic acid amplification procedures such as ligase chain reaction, ligated activated transcription, and nucleic acid sequence-based amplification may be used. The cloning procedures may involve excision and isolation of a desired nucleic acid fragment comprising the nucleic acid sequence encoding the polypeptide, insertion of the fragment into a vector molecule, and incorporation of the recombinant vector into a Bacillus cell where clones of the nucleic acid sequence will be replicated. The nucleic acid sequence may be of genomic, cDNA, RNA, semi-synthetic, synthetic origin, or any combinations thereof.

An isolated nucleic acid sequence encoding an enzyme may be manipulated in a variety of ways to provide for expression of the enzyme. Manipulation of the nucleic acid sequence prior to its insertion into a construct or vector may be desirable or necessary depending on the expression vector or *Bacillus* host cell. The techniques for modifying nucleic acid sequences utilizing cloning methods are well known in the art. It will be understood that the nucleic acid sequence may also be manipulated in vivo in the host cell using methods well known in the art.

A number of enzymes are involved in the biosynthesis of hyaluronic acid. These enzymes include hyaluronan synthase, UDP-glucose 6-dehydrogenase, UDP-glucose pyrophosphorylase, UDP-N-acetylglucosamine pyrophosphorylase, glucose-6-phosphate isomerase, hexokinase, phosphoglucomutase, amidotransferase, mutase, and acetyl transferase. Hyaluronan synthase is the key enzyme in the production of hyaluronic acid.

"Hyaluronan synthase" is defined herein as a synthase that catalyzes the elongation of a hyaluronan chain by the addition of GlcUA and GlcNAc sugar precursors. The amino acid sequences of streptococcal hyaluronan synthases, vertebrate hyaluronan synthases, and the viral hyaluronan synthase are distinct from the Pasteurella hyaluronan synthase, and have been proposed for classification as Group I and Group II hyaluronan synthases, the Group I hyaluronan synthases including Streptococcal hyaluronan synthases (DeAngelis, 1999). For production of hyaluronan in *Bacillus* host cells, hyaluronan synthases of a eukaryotic origin, such as mammalian hyaluronan synthases, are less preferred.

The hyaluronan synthase encoding sequence may be any nucleic acid sequence capable of being expressed in a *Bacillus* host cell. The nucleic acid sequence may be of any origin. Preferred hyaluronan synthase genes include any of either Group I or Group II, such as the Group I hyaluronan synthase genes from *Streptococcus equisimilis,*

*Streptococcus pyogenes, Streptococcus uberis,* and *Streptococcus equi* subsp. *zooepidemicus,* or the Group II hyaluronan synthase genes of *Pasturella multocida.*

Constructs whereby precursor sugars of hyaluronan are supplied to the host cell are preferably in producing the HA of the invention, either to the culture medium, or by being encoded by endogenous genes, by non-endogenous genes, or by a combination of endogenous and non-endogenous genes in the *Bacillus* host cell. The precursor sugar may be D-glucuronic acid or N-acetyl-glucosamine.

In the methods of the present invention, the nucleic acid construct may further comprise one or more genes encoding enzymes in the biosynthesis of a precursor sugar of a hyaluronan. Alternatively, the *Bacillus* host cell may further comprise one or more second nucleic acid constructs comprising one or more genes encoding enzymes in the biosynthesis of the precursor sugar. Hyaluronan production is improved by the use of constructs with a nucleic acid sequence or sequences encoding a gene or genes directing a step in the synthesis pathway of the precursor sugar of hyaluronan. By "directing a step in the synthesis pathway of a precursor sugar of hyaluronan" is meant that the expressed protein of the gene is active in the formation of N-acetyl-glucosamine or D-glucuronic acid, or a sugar that is a precursor of either of N-acetyl-glucosamine and D-glucuronic acid.

In a preferred method for supplying precursor sugars, constructs are provided for improving hyaluronan production in a host cell having a hyaluronan synthase, by culturing a host cell having a recombinant construct with a heterologous promoter region operably linked to a nucleic acid sequence encoding a gene directing a step in the synthesis pathway of a precursor sugar of hyaluronan. In a preferred method the host cell also comprises a recombinant construct having a promoter region operably linked to a hyaluronan synthase, which may use the same or a different promoter region than the nucleic acid sequence to a synthase involved in the biosynthesis of N-acetyl-glucosamine. In a further preferred embodiment, the host cell may have a recombinant construct with a promoter region operably linked to different nucleic acid sequences encoding a second gene involved in the synthesis of a precursor sugar of hyaluronan.

Thus, the present invention also relates to constructs for improving hyaluronan production by the use of constructs with a nucleic acid sequence encoding a gene directing a step in the synthesis pathway of a precursor sugar of hyaluronan. The nucleic acid sequence to the precursor sugar may be expressed from the same or a different promoter as the nucleic acid sequence encoding the hyaluronan synthase.

The genes involved in the biosynthesis of precursor sugars for the production of hyaluronic acid include a UDP-glucose 6-dehydrogenase gene, UDP-glucose pyrophosphorylase gene, UDP-N-acetylglucosamine pyrophosphorylase gene, glucose-6-phosphate isomerase gene, hexokinase gene, phosphoglucomutase gene, amidotransferase gene, mutase gene, and acetyl transferase gene.

In a cell containing a hyaluronan synthase, any one or combination of two or more of hasB, hasC and hasD, or the homologs thereof, such as the *Bacillus subtilis* tuaD, gtaB, and gcaD, respectively, as well as hasE, may be expressed to increase the pools of precursor sugars available to the hyaluronan synthase. The *Bacillus subtilis* genome is described in Kunst, et al., Nature 390, 249-256, "The complete genome sequence of the Gram-positive bacterium Bacillus subtilis" (20 November 1997). In some instances, such as where the host cell does not have a native hyaluronan synthase activity, the construct may include the hasA gene.

The nucleic acid sequence encoding the biosynthetic enzymes may be native to the host cell, while in other cases heterologous sequence may be utilized. If two or more genes are expressed they may be genes that are associated with one another in a native operon, such as the genes of the HAS operon of *Streptococcus equisimilis,* which comprises hasA, hasB, hasC and hasD. In other instances, the use of some combination of the precursor gene sequences may be desired, without each element of the operon included. The use of some genes native to the host cell, and others which are exogenous may also be preferred in other cases. The choice will depend on the available pools of sugars in a given host cell, the ability of the cell to accommodate overproduction without interfering with other functions of the host cell, and whether the cell regulates expression from its native genes differently than exogenous genes.

As one example, depending on the metabolic requirements and growth conditions of the cell, and the available precursor sugar pools, it may be desirable to increase the production of N-acetyl-glucosamine by expression of a nucleic acid sequence encoding UDP-N-acetylglucosamine pyrophosphorylase, such as the hasD gene, the *Bacillus* gcaD gene, and homologs thereof. Alternatively, the precursor sugar may be D-glucuronic acid. In one such embodiment, the nucleic acid sequence encodes UDP-glucose 6-dehydrogenase. Such nucleic acid sequences include the *Bacillus* tuaD gene, the hasB gene of *Streptococcus,* and homologs thereof. The nucleic acid sequence may also encode UDP-glucose pyrophosphorylase, such as in the *Bacillus* gtaB gene, the hasC gene of *Streptococcus,* and homologues thereof. In the methods of the present invention, the UDP-glucose 6-dehydrogenase gene may be a hasB gene or tuaD gene; or homologues thereof.

In the present invention it is envisioned that the hyaluronan synthase gene and the one or more genes encoding a precursor sugar are under the control of the same promoter. Alternatively, the one or more genes encoding a precursor sugar are under the control of the same promoter but a different promoter driving the hyaluronan synthase gene. A further alternative is that the hyaluronan synthase gene and each of the genes encoding a precursor sugar are under the control of different promoters. In a preferred embodiment, the hyaluronan synthase gene and the one or more genes encoding a precursor sugar are under the control of the same promoter.

The present invention also relates to a nucleic acid construct comprising an isolated nucleic acid sequence encoding a hyaluronan synthase operon comprising a hyaluronan synthase gene and a UDP-glucose 6-dehydrogenase gene, and optionally one or more genes selected from the group consisting of a UDP-glucose pyrophosphorylase gene, UDP-N-acetylglucosamine pyrophosphorylase gene, and glucose-6-phosphate isomerase gene.

In some cases the host cell will have a recombinant construct with a heterologous promoter region operably linked to a nucleic acid sequence encoding a gene directing a step in the synthesis pathway of a precursor sugar of hyaluronan, which may be in concert with the expression of hyaluronan synthase from a recombinant construct. The hyaluronan synthase may be expressed from the same or a different promoter region than the nucleic acid sequence encoding an enzyme involved in the biosynthesis of the precursor. In another preferred embodiment, the host cell may have a recombinant construct with a promoter region operably linked to a different nucleic acid sequence encoding a second gene involved in the synthesis of a precursor sugar of hyaluronan.

The nucleic acid sequence encoding the enzymes involved in the biosynthesis of the precursor sugar(s) may be expressed from the same or a different promoter as the nucleic acid sequence encoding the hyaluronan synthase. In the former sense, "artificial operons" are constructed, which may mimic the operon of *Streptococcus equisimilis* in having each hasA, hasB, hasC and hasD, or homologs thereof, or, alternatively, may utilize less than the full complement present in the Streptococcus equisimilis operon. The artificial operons" may also comprise a glucose-6-phosphate isomerase gene (hasE) as well as one or more genes selected from the group consisting of a hexokinase gene, phosphoglucomutase gene, amidotransferase gene, mutase gene, and acetyl transferase gene. In the artificial operon, at least one of the elements is heterologous to one other of the elements, such as the promoter region being heterologous to the encoding sequences.

In a preferred embodiment, the nucleic acid construct comprises hasA, tuaD, and gtaB. In another preferred embodiment, the nucleic acid construct comprises hasA, tuaD, gtaB, and gcaD. In another preferred embodiment, the nucleic acid construct comprises hasA and tuaD. In another preferred embodiment, the nucleic acid construct comprises hasA. In another preferred embodiment, the nucleic acid construct comprises hasA, tuaD, gtaB, gcaD, and hasE. In another preferred embodiment, the nucleic acid construct comprises hasA, hasB, hasC, and hasD. In another preferred embodiment, the nucleic acid construct comprises hasA, hasB, hasC, hasD, and hasE. Based on the above preferred embodiments, the genes noted can be replaced with homologs thereof.

In the methods of the present invention, the nucleic acid constructs comprise a hyaluronan synthase encoding sequence operably linked to a promoter sequence foreign to the hyaluronan synthase encoding sequence. The promoter sequence may be, for example, a single promoter or a tandem promoter.

"Promoter" is defined herein as a nucleic acid sequence involved in the binding of RNA polymerase to initiate transcription of a gene. "Tandem promoter" is defined herein as two or more promoter sequences each of which is operably linked to a coding sequence and mediates the transcription of the coding sequence into mRNA. "Operably linked" is defined herein as a configuration in which a control sequence, e.g., a promoter sequence, is appropriately placed at a position relative to a coding sequence such that the control sequence directs the production of a polypeptide encoded by the coding sequence. As noted earlier, a "coding sequence" is defined herein as a nucleic acid sequence which is transcribed into mRNA and translated into a polypeptide when placed under the control of the appropriate control sequences. The boundaries of the coding sequence are generally determined by a ribosome binding site located just upstream of the open reading frame at the 5' end of the mRNA and a transcription terminator sequence located just downstream of the open reading frame at the 3' end of the mRNA. A coding sequence can include, but is not limited to, genomic DNA, cDNA, semisynthetic, synthetic, and recombinant nucleic acid sequences.

In a preferred embodiment, the promoter sequences may be obtained from a bacterial source. In a more preferred embodiment, the promoter sequences may be obtained from a gram positive bacterium such as a *Bacillus* strain, e.g., *Bacillus agaradherens, Bacillus alkalophilus, Bacillus amyloliquefaciens, Bacillus brevis, Bacillus circulans, Bacillus clausii, Bacillus coagulans, Bacillus firmus, Bacillus lautus, Bacillus lentus, Bacillus licheniformis, Bacillus megaterium, Bacillus pumilus, Bacillus stearothermophilus, Bacillus subtilis,* or *Bacillus thuringiensis;* or a *Streptomyces* strain, e.g., *Streptomyces lividans* or *Streptomyces murinus;* or from a gram negative bacterium, e.g., *E. coli or Pseudomonas sp.*

Examples of suitable promoters for directing the transcription of a nucleic acid sequence in the methods of the present invention are the promoters obtained from the *E. coli* lac operon, *Streptomyces coelicolor* agarase gene (dagA), *Bacillus lentus* or *Bacillus clausii* alkaline protease gene (aprH), *Bacillus licheniformis* alkaline protease gene (subtilisin Carlsberg gene), *Bacillus subtilis* levansucrase gene (sacB), *Bacillus subtilis* alpha-amylase gene (amyE), *Bacillus licheniformis* alpha-amylase gene (amyL), *Bacillus stearothermophilus* maltogenic amylase gene (amyM), *Bacillus amyloliquefaciens* alpha-amylase gene (amyQ), *Bacillus licheniformis* penicillinase gene (penP), *Bacillus subtilis* xylA and xylB genes, *Bacillus thuringiensis* subsp. *tenebrionis* CryIIIA gene (cryIIIA) or portions thereof, prokaryotic beta-lactamase gene (VilIa-Kamaroff et al., 1978, Proceedings of the National Academy of Sciences USA 75:3727-3731). Other examples are the promoter of the spo1 bacterial phage promoter and the tac promoter (DeBoer et al., 1983, Proceedings of the National Academy of Sciences USA 80:21-25). Further promoters are described in "Useful proteins from recombinant bacteria" in Scientific American, 1980, 242:74-94; and in Sambrook, Fritsch, and Maniatus, 1989, Molecular Cloning, A Laboratory Manual, 2d edition, Cold Spring Harbor, New York.

The promoter may also be a "consensus" promoter having the sequence TTGACA for the "-35" region and TATAAT for the "-10" region. The consensus promoter may be obtained from any promoter which can function in a *Bacillus* host cell. The construction of a "consensus" promoter may be accomplished by site-directed mutagenesis to create a promoter which conforms more perfectly to the established consensus sequences for the " 10" and "-35" regions of the vegetative "sigma A-type" promoters for *Bacillus subtilis* (Voskuil et al., 1995, Molecular Microbiology 17: 271-279).

In a preferred embodiment, the "consensus" promoter is obtained from a promoter obtained from the E. coli lac operon, Streptomyces coelicolor agarase gene (dagA), Bacillus clausii or Bacillus lentus alkaline protease gene (aprH), Bacillus licheniformis alkaline protease gene (subtilisin Carlsberg gene), Bacillus subtilis levansucrase gene (sacB), Bacillus subtilis alpha-amylase gene (amyE), Bacillus licheniformis alpha-amylase gene (amyL), Bacillus stearothermophilus maltogenic amylase gene (amyM), Bacillus amyloliquefaciens alpha-amylase gene (amyQ), Bacillus licheniformis penicillinase gene (penP), Bacillus subtilis xylA and xylB genes, Bacillus thuringiensis subsp. tenebrionis CryIIIA gene (cryIIIA) or portions thereof, or prokaryotic beta-lactamase gene spo1 bacterial phage promoter. In a more preferred embodiment, the "consensus" promoter is obtained from Bacillus amyloliquefaciens alpha-amylase gene (amyQ).

Widner, et al., United States Patent Nos. 6,255,076 and 5,955,310, describe tandem promoters and constructs and methods for use in expression in Bacillus cells, including the short consensus amyQ promoter (also called scBAN). The use of the cryIIIA stabilizer sequence, and constructs using the sequence, for improved production in Bacillus are also described therein.

Each promoter sequence of the tandem promoter may be any nucleic acid sequence which shows transcriptional activity in the Bacillus cell of choice including a mutant, truncated, and hybrid promoter, and may be obtained from genes encoding extracellular or intracellular polypeptides either homologous or heterologous to the Bacillus cell. Each promoter sequence may be native or foreign to the nucleic acid sequence encoding the polypeptide and native or foreign to the Bacillus cell. The promoter sequences may be the same promoter sequence or different promoter sequences.

The two or more promoter sequences of the tandem promoter may simultaneously promote the transcription of the nucleic acid sequence. Alternatively, one or more of the promoter sequences of the tandem promoter may promote the transcription of the nucleic acid sequence at different stages of growth of the Bacillus cell.

In a preferred embodiment, the tandem promoter contains at least the amyQ promoter of the Bacillus amyloliquefaciens alpha-amylase gene. In another preferred embodiment, the tandem promoter contains at least a "consensus" promoter having the sequence TTGACA for the "-35" region and TATAAT for the "-10" region. In another preferred embodiment, the tandem promoter contains at least the amyL promoter of the Bacillus licheniformis alpha-amylase gene. In another preferred embodiment, the tandem promoter contains at least the cryIIIA promoter or portions thereof (Agaisse and Lereclus, 1994, Molecular Microbiology 13: 97-107).

In a more preferred embodiment, the tandem promoter contains at least the amyL promoter and the cryIIIA promoter. In another more preferred embodiment, the tandem promoter contains at least the amyQ promoter and the cryIIIA promoter. In another more preferred embodiment, the tandem promoter contains at least a "consensus" promoter having the sequence TTGACA for the "-35" region and TATAAT for the "-10" region and the cryIIIA promoter. In another more preferred embodiment, the tandem promoter contains at least two copies of the amyL promoter. In another more preferred embodiment, the tandem promoter contains at least two copies of the amyQ promoter. In another more preferred embodiment, the tandem promoter contains at least two copies of a "consensus" promoter having the sequence TTGACA for the "-35" region and TATAAT for the "-10" region. In another more preferred embodiment, the tandem promoter contains at least two copies of the cryIIIA promoter.

"An mRNA processing/stabilizing sequence" is defined herein as a sequence located downstream of one or more promoter sequences and upstream of a coding sequence to which each of the one or more promoter sequences are operably linked such that all mRNAs synthesized from each promoter sequence may be processed to generate mRNA transcripts with a stabilizer sequence at the 5' end of the transcripts. The presence of such a stabilizer sequence at the 5' end of the mRNA transcripts increases their half-life (Agaisse and Lereclus, 1994, supra, Hue et al., 1995, Journal of Bacteriology 177: 3465-3471). The mRNA processing/stabilizing sequence is complementary to the 3' extremity of a bacterial 16S ribosomal RNA. In a preferred embodiment, the mRNA processing/stabilizing sequence generates essentially single-size transcripts with a stabilizing sequence at the 5' end of the transcripts. The mRNA processing/ stabilizing sequence is preferably one, which is complementary to the 3' extremity of a bacterial 16S ribosomal RNA. See, U.S. Patent Nos. 6,255,076 and 5,955,310.

In a more preferred embodiment, the mRNA processing/stabilizing sequence is the Bacillus thuringiensis cryIIIA mRNA processing/stabilizing sequence disclosed in WO 94/25612 and Agaisse and Lereclus, 1994, supra, or portions thereof which retain the mRNA processing/stabilizing function. In another more preferred embodiment, the mRNA processing/stabilizing sequence is the Bacillus subtilis SP82 mRNA processing/stabilizing sequence disclosed in Hue et al., 1995, supra, or portions thereof which retain the mRNA processing/stabilizing function.

When the cryIIIA promoter and its mRNA processing/stabilizing sequence are employed in the methods of the present invention, a DNA fragment containing the sequence disclosed in WO 94/25612 and Agaisse and Lereclus, 1994, supra, or portions thereof which retain the promoter and mRNA processing/stabilizing functions, may be used. Furthermore, DNA fragments containing only the cryIIIA promoter or only the cryIIIA mRNA processing/stabilizing sequence may be prepared using methods well known in the art to construct various tandem promoter and mRNA processing/stabilizing sequence combinations. In this embodiment, the cryIIIA promoter and its mRNA processing/stabilizing sequence are preferably placed downstream of the other promoter sequence(s) constituting the tandem promoter and upstream of the coding sequence of the gene of interest.

The isolated nucleic acid sequence encoding the desired enzyme(s) involved in hyaluronic acid production may then be further manipulated to improve expression of the nucleic acid sequence. Expression will be understood to include any step involved in the production of the polypeptide including, but not limited to, transcription, post-transcriptional modification, translation, post-translational modification, and secretion. The techniques for modifying nucleic acid sequences utilizing cloning methods are well known in the art.

A nucleic acid construct comprising a nucleic acid sequence encoding an enzyme may be operably linked to one or more control sequences capable of directing the expression of the coding sequence in a Bacillus cell under conditions compatible with the control sequences.

The term "control sequences" is defined herein to include all components which are necessary or advantageous for expression of the coding sequence of a nucleic acid sequence. Each control sequence may be native or foreign to the nucleic acid sequence encoding the enzyme. In addition to promoter sequences described above, such control sequences include, but are not limited to, a leader, a signal sequence, and a transcription terminator. At a minimum, the control sequences include a promoter, and transcriptional and translational stop signals. The control sequences may be provided with linkers for the purpose of introducing specific restriction sites facilitating ligation of the control sequences with the coding region of the nucleic acid sequence encoding an enzyme.

The control sequence may also be a suitable transcription terminator sequence, a sequence recognized by a Bacillus cell to terminate transcription. The terminator sequence is operably linked to the 3' terminus of the nucleic acid sequence encoding the enzyme or the last enzyme of an operon. Any terminator which is functional in the Bacillus cell of choice may be used in the present invention.

The control sequence may also be a suitable leader sequence, a nontranslated region of a mRNA which is important for translation by the Bacillus cell. The leader sequence is operably linked to the 5' terminus of the nucleic acid sequence encoding the enzyme. Any leader sequence which is functional in the Bacillus cell of choice may be used in the present invention.

The control sequence may also be a signal peptide coding region, which codes for an amino acid sequence linked to the amino terminus of a polypeptide which can direct the expressed polypeptide into the cell's secretory pathway. The signal peptide coding region may be native to the polypeptide or may be obtained from foreign sources. The 5' end of the coding sequence of the nucleic acid sequence may inherently contain a signal peptide coding region naturally linked in translation reading frame with the segment of the coding region which encodes the secreted polypeptide. Alternatively, the 5' end of the coding sequence may contain a signal peptide coding region which is foreign to that portion of the coding sequence which encodes the secreted polypeptide. The foreign signal peptide coding region may be required where the coding sequence does not normally contain a signal peptide coding region. Alternatively, the foreign signal peptide coding region may simply replace the natural signal peptide coding region in order to obtain enhanced secretion of the polypeptide relative to the natural signal peptide coding region normally associated with the coding sequence. The signal peptide coding region may be obtained from an amylase or a protease gene from a Bacillus species. However, any signal peptide coding region capable of directing the expressed polypeptide into the secretory pathway of a Bacillus cell of choice may be used in the present invention.

An effective signal peptide coding region for Bacillus cells is the signal peptide coding region obtained from the maltogenic amylase gene from Bacillus NCIB 11837, the Bacillus stearothermophilus alpha-amylase gene, the Bacillus licheniformis subtilisin gene, the Bacillus licheniformis beta-lactamase gene, the Bacillus stearothermophilus neutral proteases genes (nprT, nprS, nprM), and the Bacillus subtilis prsA gene. Further signal peptides are described by Simonen and Palva, 1993, Microbiological Reviews 57:109-137.

The control sequence may also be a propeptide coding region that codes for an amino acid sequence positioned at the amino terminus of a polypeptide. The resultant polypeptide is known as a proenzyme or propolypeptide (or a zymogen in some cases). A propolypeptide is generally inactive and can be converted to a mature active polypeptide by catalytic or autocatalytic cleavage of the propeptide from the propolypeptide. The propeptide coding region may be obtained from the genes for Bacillus subtilis alkaline protease (aprE) and Bacillus subtilis neutral protease (nprT).

Where both signal peptide and propeptide regions are present at the amino terminus of a polypeptide, the propeptide region is positioned next to the amino terminus of a polypeptide and the signal peptide region is positioned next to the amino terminus of the propeptide region.

It may also be desirable to add regulatory sequences which allow the regulation of the expression of the polypeptide relative to the growth of the host cell. Examples of regulatory systems are those which cause the expression of the gene to be turned on or off in response to a chemical or physical stimulus, including the presence of a regulatory compound. Regulatory systems in prokaryotic systems include the lac, tac, and trp operator systems.

Production

In the methods of the present invention, the host cells are cultivated in a nutrient medium suitable for production of the hyaluronic acid using methods known in the art. For example, the cell may be cultivated by shake flask cultivation, small-scale or large-scale fermentation (including continuous, batch, fed-batch, or solid state fermentations) in laboratory or industrial fermentors performed in a suitable medium and under conditions allowing the enzymes involved in hyaluronic acid synthesis to be expressed and the hyaluronic acid to be isolated. The cultivation takes place in a suitable nutrient medium comprising carbon and nitrogen sources and inorganic salts, using procedures known in the art. Suitable media are available from commercial suppliers or may be prepared according to published compositions (e.g., in catalogues of the American Type Culture Collection). The secreted hyaluronic acid can be recovered directly from the medium.

The resulting hyaluronic acid may be isolated by methods known in the art. For example, the hyaluronic acid may be isolated from the nutrient medium by conventional procedures including, but not limited to, centrifugation, filtration, extraction, spray-drying, evaporation, or precipitation. The isolated hyaluronic acid may then be further purified by a variety of procedures known in the art including, but not limited to, chromatography (e.g., ion exchange, affinity, hydrophobic, chromatofocusing, and size exclusion), electrophoretic procedures (e.g., preparative isoelectric focusing), differential solubility (e.g., ammonium sulfate precipitation), or extraction (see, e.g., Protein Purification, J.-C. Janson and Lars Ryden, editors, VCH Publishers, New York, 1989).

## DETAILED DESCRIPTION

Hyaluronic Acid

"Hyaluronic acid" is defined herein as an unsulphated glycosaminoglycan composed of repeating disaccharide units of N-acetylglucosamine (GlcNAc) and glucuronic acid (GlcUA) linked together by alternating beta-1,4 and beta-1,3 glycosidic bonds. Hyaluronic acid is also known as hyaluronan, hyaluronate, or HA. The terms hyaluronan and hyaluronic acid are used interchangeably herein.

Rooster combs are a significant commercial source for hyaluronan. Microorganisms are an alternative source. U.S. Patent No. 4,801,539 discloses a fermentation method for preparing hyaluronic acid involving a strain of *Streptococcus zooepidemicus* with reported yields of about 3.6 g of hyaluronic acid per liter. European Patent No. EP0694616 discloses fermentation processes using an improved strain of *Streptococcus zooepidemicus* with reported yields of about 3.5 g of hyaluronic acid per liter. As disclosed in WO 03/054163 (Novozymes), which is incorporated herein in its entirety, hyaluronic acid or salts thereof may be recombinantly produced, e.g., in a Gram-positive *Bacillus* host.

Hyaluronan synthases have been described from vertebrates, bacterial pathogens, and algal viruses (DeAngelis, P. L., 1999, Cell. Mol. Life Sci. 56: 670-682). WO 99/23227 discloses a Group I hyaluronate synthase from *Streptococcus equisimilis.* WO 99/51265 and WO 00/27437 describe a Group II hyaluronate synthase from *Pasturella multocida.* Ferretti *et al.* disclose the hyaluronan synthase operon of *Streptococcus pyogenes,* which is composed of three genes, *hasA, hasB, and hasC,* that encode hyaluronate synthase, UDP glucose dehydrogenase, and UDP-glucose pyrophosphorylase, respectively (Proc. Natl. Acad. Sci. USA. 98, 4658-4663, 2001). WO 99/51265 describes a nucleic acid segment having a coding region for a *Streptococcus equisimilis* hyaluronan synthase.

Since the hyaluronan of a recombinant *Bacillus* cell is expressed directly to the culture medium, a simple process may be used to isolate the hyaluronan from the culture medium. First, the *Bacillus* cells and cellular debris are physically removed from the culture medium. The culture medium may be diluted first, if desired, to reduce the viscosity of the medium. Many methods are known to those skilled in the art for removing cells from culture medium, such as centrifugation or microfiltration. If desired, the remaining supernatant may then be filtered, such as by ultrafiltration, to concentrate and remove small molecule contaminants from the hyaluronan. Following removal of the cells and cellular debris, a simple precipitation of the hyaluronan from the medium is performed by known mechanisms. Salt, alcohol, or combinations of salt and alcohol may be used to precipitate the hyaluronan from the filtrate. Once reduced to a precipitate, the hyaluronan can be easily isolated from the solution by physical means. The hyaluronan may be dried or concentrated from the filtrate solution by using evaporative techniques known to the art, such as spray drying.

The first aspect of the invention relates to a product comprising hyaluronic acid or a salt thereof, which product is dried and agglomerated as defined herein.

Host Cells

A preferred embodiment relates to the product of the first aspect, wherein the hyaluronic acid or salt thereof is recombinantly produced, preferably by a Gram-positive bacterium or host cell, more preferably by a bacterium of the genus *Bacillus.*

The host cell may be any *Bacillus* cell suitable for recombinant production of hyaluronic acid. The *Bacillus* host cell may be a wild-type *Bacillus* cell or a mutant thereof. *Bacillus* cells useful in the practice of the present invention include, but are not limited to, *Bacillus agaraderhens, Bacillus alkalophilus, Bacillus amyloliquefaciens, Bacillus brevis, Bacillus circulans, Bacillus clausii, Bacillus coagulans, Bacillus firmus, Bacillus lautus, Bacillus lentus, Bacillus licheniformis, Bacillus megaterium, Bacillus pumilus, Bacillus stearothermophilus, Bacillus subtilis,* and *Bacillus thuringiensis* cells. Mutant *Bacillus subtilis* cells particularly adapted for recombinant expression are described in WO 98/22598. Non-encapsulating *Bacillus* cells are particularly useful in the present invention.

In a preferred embodiment, the Bacillus host cell is a *Bacillus amyloliquefaciens, Bacillus clausii, Bacillus lentus, Bacillus licheniformis, Bacillus stearothermophilus* or *Bacillus subtilis* cell. In a more preferred embodiment, the *Bacillus* cell is a *Bacillus amyloliquefaciens* cell. In another more preferred embodiment, the *Bacillus* cell is a *Bacillus clausii* cell. In another more preferred embodiment, the *Bacillus* cell is a *Bacillus lentus* cell. In another more preferred embodiment, the *Bacillus* cell is a *Bacillus licheniformis* cell. In another more preferred embodiment, the *Bacillus* cell is a *Bacillus subtilis* cell. In a most preferred embodiment, the *Bacillus* host cell is *Bacillus subtilis* A164Δ5 (see U.S. Patent No. 5,891,701) or *Bacillus subtilis* 168Δ4.

Transformation of the Bacillus host cell with a nucleic acid construct of the present invention may, for instance, be effected by protoplast transformation (see, e.g., Chang and Cohen, 1979, Molecular General Genetics 168: 111-115), by using competent cells (see, e.g., Young and Spizizen, 1961, Journal of Bacteriology 81: 823-829, or Dubnau and Davidoff-Abelson, 1971, Journal of Molecular Biology 56: 209-221), by electroporation (see, e.g., Shigekawa and Dower, 1988, Biotechniques 6: 742-751), or by conjugation (see, e.g., Koehler and Thorne, 1987, Journal of Bacteriology 169: 5271-5278).

Molecular weight

The level of hyaluronic acid may be determined according to the modified carbazole method (Bitter and Muir, 1962, Anal Biochem. 4: 330-334). Moreover, the average molecular weight of the hyaluronic acid may be determined using standard methods in the art, such as those described by Ueno et al., 1988, Chem. Pharm. Bull. 36, 4971-4975; Wyatt, 1993, Anal. Chim. Acta 272: 1-40; and Wyatt Technologies, 1999, "Light Scattering University DAWN Course Manual" and "DAWN EOS Manual" Wyatt Technology Corporation, Santa Barbara, California.

In a preferred embodiment, the hyaluronic acid obtained by the methods of the present invention has a molecular weight of about 10,000 to about 10,000,000 Da. In a more preferred embodiment, the hyaluronic acid obtained by the methods of the present invention has a molecular weight of about 25,000 to about 5,000,000 Da. In a most preferred embodiment, the hyaluronic acid obtained by the methods of the present invention has a molecular weight of about 50,000 to about 3,000,000 Da.

A preferrred embodiment relates to the product of the first aspect, wherein the hyaluronic acid or salt thereof has a molecular weight in the range of between 300,000 and 3,000,000; preferably in the range of between 400,000 and 2,500,000; more preferably in the range of between 500,000 and 2,000,000; and most preferably in the range of between 600,000 and 1,800,000 Da.

Where recombinantly produced hyaluronic acid or salt thereof is used in the methods of the invention to manufacture the products or compositions of the invention, it may be advantageous for some applications to first reduce the average molecular weight of the hyaluronic acid or salt thereof. For instance, it has been stated by several manufacturers of so-called low-molecular weight fractions of hyaluronic acid, that it is capable of penetrating the skin barrier to reestablish the natural content of hyaluronic acid in the skin, therefore such fractions are particularly suitable for cosmetic compositions sold as anti-skin-ageing and anti-wrinkle agents. For food applications, low MW hyaluronic acid has been shown to penetrate the gastrointestinal barrier, thereby increasing its bioavailability. Finally, low MW hyaluronic acid exhibits anti-inflammatory effect and have potential applications in the treatment of inflammatory diseases. A reduction of the average molecular weight of a hyaluronic acid or salt thereof may be achieved by standard methods in the art, such as, simple heat treatment, enzymatic degradation, ultrasound sonication, or acid hydrolysis. See, e.g., US patent 6,020,484, which describes an ultrasonication technique of HA including NaOCl as additive, and T. Miyazaki et al. (2001) Polymer Degradation and Stability, 74: 77-85.

Accordingly, a preferred embodiment relates to the product of the first aspect, wherein the hyaluronic acid or salt thereof has a low average molecular weight in the range of between 10,000 and 800,000 Da; preferably in the range of between 20,000 and 600,000 Da; more preferably in the range of between 30,000 and 500,000 Da; even more preferably in the range of between 40,000 and 400,000 Da; and most preferably in the range of between 50,000 and 300,000 Da.

Salts and crosslinked HA

A preferred embodiment relates to a product of the first aspect, which comprises an inorganic salt of hyaluronic acid, preferably sodium hyaluronate, potassium hyaluronate, ammonium hyaluronate, calcium hyaluronate, magnesium hyaluronate, zinc hyaluronate, or cobalt hyaluronate.

The preparation of a crosslinked HA or salt thereof, which is prepared by crosslinking HA with a polyfunctional epoxy compound is disclosed in EP 0 161 887 B1. Total or partial crosslinked esters of HA with an aliphatic alcohol, and salts of such partial esters with inorganic or organic bases, are disclosed in US 4,957,744. Other ways of cross-linking HA are disclosed in U.S. Patent Nos. 5,616,568, 5,652,347, and 5,874,417. Crosslinked HA may also be prepared by treating HA with boric acid, as follows:

Dried sodium hyaluronate (Na-HA, 203 mg), recombinantly produced in a *Bacillus subtilis* by fermentation (WO 03/054163; Novozymes), was dissolved into 0.2 M NaOH to give a 4% solution. Boric acid (35 mg (approx. 1 equivalent of HA disaccharide) was added and the sample was stirred at room temperature for 1.5 h, and then stored at 5°C for ca. 2.5 days. A control sample was prepared in parallel exactly as described above, but without boric acid.

The viscosity of the resulting HA-borate hydrogel was measured at 25°C using a Carrimed CSL controlled stress rheometer (cone geometry: 6 cm, 2°). The viscosity depended on the shear rate and increased at least 4-fold (from 4.2- to 8.4 fold) in the HA-borate hydrogel as compared to the control sample, indicating formation of a cross-linked network.

New peaks at 1200 and 945 cm-1 were observed on the FT-IR spectrum of the HA-borate hydrogel, when compared to a standard spectrum of Na-HA, corresponding to the presence of newly formed borate esters in the crosslinked HA-borate hydrogel.

Accordingly, a preferred embodiment relates to the product of the first aspect, which comprises crosslinked hyaluronic acid or salt thereof, preferably the hyaluronic acid is crosslinked with boric acid, and more preferably the crosslinked hyaluronic acid comprises borate esters.

Determination of moisture

The moisture content of a dried product powder according to the invention is the loss in weight, expressed as a percentage, after drying the powder at 102°C $\pm$2°C to a constant weight. An empty glass weighing dish with a ground lid is dried in the oven, then cooled and weighed on an analytical balance with a sensitivity of at least 0.1 mg. Approximately 3 g dried product powder is placed in the dish and weighed. The dish with the powder is placed without the lid in the oven and dried for 2 hours at a temperature of 102°C $\pm$2°C; then it is placed in a desiccator and cooled to room temperature before it is weighed again. The dish with the powder is placed without the lid in the oven to dry for 1 more hour, and then cooled and weighed as already described; this is repeated until the weight remains constant, *i.e.,* until two successive weighings do not differ by more than 0.5 mg.

The percentage of moisture is then calculated as: (W2-W3)/(W2-W1)x100; where W1 is the weight of the empty dish, W2 is the weight of the dish with powder, and W3 is the weight of the dish with dried powder. The result is calculated to 2 decimal places, and the reproducibility of this method is about $\pm$0.1%.

Preferably, the dried and agglomerated product of the first aspect, comprises less than 5% moisture, preferably less than 2%, and most preferably less than 1% moisture, as determined herein.

Particle size

A preferred product of the first aspect has a particle size the 50 percentile of which, $D_{50}$, is between 10 and 1,000 microns, preferably between 100 and 1,000 microns, more preferably between 150 and 900 microns, and even more preferably between 200 and 800 microns, as determined by laser diffraction measurement of the particles suspended in isopropanol, as shown in the examples below.

In a preferred embodiment, the polydispersity of a product of the first aspect is measured as the SPAN value, which is calculated according to the following formula: SPAN = $(D_{90}-D_{10})/D_{50}$, and the SPAN value is between 1.0 and 2.5; preferably the SPAN value is between 1.2 and 2.2; more preferably the SPAN value is between 1.5 and 1.9; and most preferably the SPAN value is between 1.6 and 1.8.

Dispersibility

The dispersibility of the product of the invention is determined as follows: 0.1 g of the dried and agglomerated product is added to a 100 ml glass bottle. 50 ml of a 0.9% (w/v) sodium chloride solution is added and the bottle is sealed and inverted 180 degrees by hand repeatedly until all particles added are dispersed as single particles, *i.e.,* until no lumps are left, as determined by visual inspection, and record the time taken to perform the inversions on a data sheet. The time thus recorded is the dispersibility of the product.

A preferred product of the first aspect has a dispersibility of less than 30 minutes in an aqueous solvent, which preferably is water, preferably less than 20 minutes, more preferably less than 15 minutes, more preferably less than 10 minutes, more preferably less than 5 minutes, more preferably less than 3 minutes, more preferably less than 2 minutes, and most preferably less than 1 minute, as determined herein.

Solubility

The solubility in various solvents of a dried, or dried and aggregated, product or composition comprising hyaluronic acid according to the invention may be determined as follows:

Add 0.1 g of the hyaluronan powder to be tested to a 100ml sealable glass flask. The add 50 ml of 0.9% (w/v) sodium chloride, injection grade water, absolute ethanol, acetone or ether to the flask containing the HA sample to be tested. Seal the flask and perform 180 degrees inversions by hand until the HA goes into solution, as determined by visual inspection, and record the time taken to perform the inversions on a data sheet. The time recorded is the solubility of the sample.

A preferred product according to the first aspect has a solubility of less than 60 minutes in an aqueous solvent, preferably less than 45 minutes, more preferably less than 40 minutes, even more preferably less than 35 minutes, 30 minutes, 25 minutes, 20 minutes, 15 minutes, 10 minutes, and most preferably less than 5 minutes, as determined herein.

Other ingredients

In a preferred embodiment, the dried and agglomerated product of the invention may also comprise other ingredients, preferably one or more active ingredient, preferably one or more pharmacologically active substance, and also preferably a water-soluble excipient, such as lactose.

Non-limiting examples of an active ingredient or pharmacologically active substance which may be used in the present invention include protein and/or peptide drugs, such as, human growth hormone, bovine growth hormone, porcine growth hormone, growth homorne releasing hormone/peptide, granulocyte-colony stimulating factor, granulocyte macrophage-colony stimulating factor, macrophage-colony stimulating factor, erythropoietin, bone morphogenic protein, interferon or derivative thereof, insulin or derivative thereof, atriopeptin-III, monoclonal antibody, tumor necrosis factor, macrophage activating factor, interleukin, tumor degenerating factor, insulin-like growth factor, epidermal growth factor, tissue plasminogen activator, factor IIV, factor IIIV, and urokinase.

A water-soluble excipient my be included for the purpose of stabilizing the active ingredient(s), such excipient may include a protein, e.g., albumin or gelatin; an amino acid, such as glycine, alanine, glutamic acid, arginine, lysine and a salt thereof; carbohydrate such as glucose, lactose, xylose, galactose, fructose, maltose, saccharose, dextran, mannitol, sorbitol, trehalose and chondroitin sulphate; an inorganic salt such as phosphate; a surfactant such as TWEEN® (ICI), poly ethylene glycol, and a mixture thereof. The excipient or stabilizer may be used in an amount ranging from 0.001 to 99% by weight of the product.

Several aspects of the invention relate to various compositions and pharmaceutical comprising, amonth other constituents, an effective amount of the product as defined in the first aspect, and an active ingredient, preferably the active ingredient is a pharmacologically active agent; a pharmaceutically acceptable carrier, excipient or diluent, preferably a water-soluble excipient, and most preferably lactose.

A preferred embodiment of the invention relates to products or compositions of the invention comprised in an effervescent tablet, which may otherwise be formulated as described in the art. For instance, an effervescent tablet may comprise citric acid, sodium bicarbonate, and an oligosaccharide or other sugar. Effervescent tablets are easy to store, and with the fast-dissolving product of the present invention, they are quickly dissolved and thus provide an ideal means of oral administration.

In addition, aspects of the invention relate to articles comprising a product as defined in the first aspect or a composition as defined in the aspects and embodiments above, e.g., a cosmetic article, a sanitary article, a medical or surgical article. In a final aspect the invention relates to a medicament capsule or microcapsule comprising a product as defined in the first aspect or a composition as defined in other aspects and embodiments of the invention.

Method of Production

The present invention in another aspect provides a method of producing a product as defined in the first aspect of the invention and/or preferred embodiments thereof, the method comprising the steps of:

a) drying a product comprising hyaluronic acid or a salt thereof; and
b) agglomerating the dried product.

Spray drying and agglomerating

In a preferred embodiment of the method, the drying step is done using a spray-dryer, preferably a Two-Fluid-Nozzle (TFN) or a Rotary Atomizer, and preferably the spray-drying was done using a TFN and the following condition ranges:

| | |
|---|---|
| Inlet temperature: | 100 - 200 °C |
| Outlet temperature: | 40 - 90 °C |
| Nozzle atomization pressure: | 2 - 10 bar |
| Nozzle air temperature: | 40 - 100 °C |
| Feed temperature: | 40 - 100 °C |

Another preferred embodiment relates to the method above, wherein the agglomerating step is done using a fluidised bed; preferably wherein the fluidised bed inlet temperature is in the range of 30 - 80 °C, and wherein the aqueous solution of the powder is sprayed on the dried powder at a rate corresponding to an outlet temperature of 25 - 50 °C.

Yet another preferred embodiment relates to the method above, wherein the agglomerating step is done using a water-solvent mixture, preferably having a water-solvent ratio of between 0:100 and 50:50, and more preferably having a water:solvent ratio of between 0:100 and 20:80; preferably the solvent is an alcohol, and more preferably the solvent is iso-propanol.

It is preferred that the drying and agglomerating is done in one step, preferably using a fluidised spray-dryer.

Methods of using the product or composition

Various aspects of the invention relate to methods of performing treatment procedures, e.g., in the medical field, using a product of the first aspect, or using compositions of the invention.

One aspect relates to a method of performing procedures in ophtalmology, which comprises the use of a product as defined in the first aspect or a composition of the invention.

Another aspect relates to a method of performing procedures in the treatment of osteoarthritis, which comprises the use of a product as defined in the first aspect or a composition of the invention.

Yet another aspect relates to a method of performing procedures in the treatment of cancer, which comprises the use of a product as defined in the first aspect or a composition of the invention.

Still another aspect relates to a method of performing procedures in the treatment of hair loss or baldness, the improvement which comprises the use of a product as defined in the first aspect or a composition of the invention.

An aspect relates to a method of performing transdermal or dermal administration of a pharmacologically active agent, which comprises the use of a product as defined in the first aspect or a composition of the invention.

Another aspect relates to a method of performing dermal administration of a cosmetic, which comprises the use of a product as defined in the first aspect or a composition of the invention.

An aspect relates to an ophtamological method of using a product as defined in the first aspect or a composition of the invention.

Another aspect relates to a method of treating osteoarthritis, comprising administering an effective amount of a product as defined in the first aspect or a composition of the invention to a mammal, preferably the administering is dermal, transdermal, oral, or by injection.

An aspect relates to a method of treating a wound, comprising administering an effective amount of a product as defined in the first aspect or a composition of the invention to a mammal.

Still another aspect relates to a method of treating hair loss or baldness, comprising administering an effective amount of a product as defined in the first aspect or a composition of the invention to a mammal, preferably the administering is dermal, transdermal, oral, or by injection.

Other aspects relate to the use of a product as defined in the first aspect or a composition of the invention for the manufacture of a medicament for the treatment of osteoarthritis, a medicament for an ophtalmological treatment, a medicament for the treatment of cancer, a medicament for the treatment of a wound, a medicament for angiogenesis, or a medicament for the treatment of hair loss or baldness.

A final aspect relates to the use of a product as defined in the first aspect or a composition of the invention for the manufacture of a moisturizer.

## EXAMPLES

### Example 1

A liquid feed consisting of Sodium Hyaluronate solution containing about 8 g / litre was spray dried using two different types of atomization techniques: A) Two-Fluid-Nozzle (TFN) and B) Rotary Atomizer. Commercially available types of spray-drying techniques and equipment are envisioned as being suitable for the purposes of this invention also (See e.g. Niro Atomizers Inc).

The resulting dried powders were noticeable different. The powder produced by the TFN had a significantly smaller bulk density. The reason for this difference was revealed upon further analysis shown below.

| Atomizer (batch no.) | Particle Density (g/ml) | Solubility Time (min) | Particle Size ($D_{50}$) |
|---|---|---|---|
| A) TFN (04PBAC0016) | 0.210 | 15 | 95 |
| B) Rotary Atomizer (MAG30012) | 0.430 | 31 | 45 |

It was seen that the solubility time was significantly decreased using the TFN atomization principle, as compared to the rotary atomizer. Despite the fact that the particle size was much bigger, which normally would lead to increased solubility time. The decreased solubility time was, however, found to be due to included air in the TFN spray dried particles. This could be seen from the particle density of the TFN product, which was less than half of that of the dried product produced by the rotary atomizer.

### Example 2

A liquid feed preparation consisting of 75,000 g Sodium Hyaluronate solution, with about 8 g/litre Sodium Hyaluronate and 2400 g sodium chloride was added to a spray dryer at constant rate. The Hyaluronate had a molecular weight of about 690.000 Da. The spray dryer had a diameter of about 1.8 m and a height of about 5 m. A Two-Fluid-Nozzle (TFN) was selected for the atomization of the feed. The drying conditions were adjusted to give a dry powder batch (batch nr. 03PBAC0035-1) with the desired particle size:

| | |
|---|---|
| Inlet temperature: | 160 °C |
| Outlet temperature: | 75 °C |
| Nozzle atomization pressure: | 4 bar |
| Nozzle air temperature: | 70 °C |
| Feed temperature: | 60 °C |

The resulting particle size distribution was measured using laser diffraction on a suspension of the powder in isopropanol:

| | |
|---|---|
| $D_{10}$ : | 12 microns |
| $D_{50}$ : | 39 microns |
| $D_{90}$ : | 84 microns |
| Molecular weight: | 631.000 Da |
| SPAN: | 1.77 |

A measure of the polydispersity is the 'SPAN' value, which may be calculated according to the following formula:

$$SPAN = (D_{90} - D_{10})/D_{50}$$

The molecular weight was nearly unchanged during the drying operation, thus demonstrating the spray drying process to be very gentle despite the high temperatures applied.

**Example 3**

5 g samples of the spray-dried powder manufactured in Example 2 was agglomerated by suspending powder samples in 20 ml of each of the following solvent-water mixtures:

100 % isopropanol (IPA)
90 % isopropanol
87 % isopropanol

The suspensions were subsequently filtered and dried in vacuum. The dried filter cakes were crushed and screened through sieves to remove all particles below 150 microns and larger than 600 microns.

It was surprisingly found that the agglomerated particles thus manufactured were very fast dispersing and dissolving in saline. To investigate this phenomenon, the particle size was measured more accurately using laser diffraction on a suspension of the product in pure IPA. The particle sizes thus detemined (10, 50, and 90 percentiles) are shown in table 1 below. A significant increase in the average agglomerated particle size was observed, as the water content in the solvent-water mixture used for agglomeration was increased from 0 to 13%.

**Table** 1

| Particle size | D(v,0.1) micron | D(v,0.5) Micron | D(v,0.9) micron |
|---|---|---|---|
| 100% IPA | 14 | 60 | 242 |
| 90% IPA | 20 | 71 | 505 |
| 87% IPA | 21 | 179 | 538 |

In order to achieve a sufficiently fast dispersion it was found that the particle size should be above approximately 180 micron. Consequently, a sieve fraction from 180 - 355 micron of the batch which was agglomerated using 87 % IPA was prepared. This fraction was subsequently compared to the non-agglomerated but otherwise identical product from Example 2. The results are shown in table 2 below. Clearly, the solubility time is significantly improved by the agglomeration process.

**Table 2**

| Batch (treatment) | Dispersion time sec | Solubility time min |
|---|---|---|
| 03PBAC0035 (Non agglomerated) | > 1 minute | 14.0 |
| 03PBAC0035 (Agglomerated, 87 % IPA) | < 1 sec | 2.5 |

**Example 4**

The method described in Example 3 was used to treat the two batches described in Example 1 to see if it would be possible to modify the solubility time of a pure hyaluronate product. In table 3 below the results are shown for a sieve fraction of 250-500 micron for the agglomerated products. It is clearly seen that the solubility time may be significantly reduced by combining the methods of Example 1 and 3.

**Table 3**

| Atomizer (batch no.) | Solubility Time, Non agglomerated (min) | Solubility Time agglomerated (min) |
|---|---|---|
| A) TFN (04PBAC0016) | 15 | 9 |
| B) Rotary Atomizer (MAG30012) | 31 | 17 |

**Example 5**

The powder manufactured in Example 1 was treated in following way: About 100 g dried powder was added to a Strea laboratory fluid bed. The inlet temperature was 55 °C. A 1 % aqueous solution of the powder manufactured in Example 1 was then sprayed on the dried powder at a rate corresponding to an outlet temperature of 35 °C. The resulting formed agglomerates were tested for solubility, and they showed a remarkably improved solubility when compared to the untreated powder.

**Example 6**

A liquid feed preparation consisting of 75,000 g Sodium Hyaluronate solution and 2,400 g sodium chloride were added to a fluidized spray dryer (FSD) at constant rate. The FSD spray dryer was equipped with an integrated fluid-bed placed beneath the spray drying tower. The drying air with entrained fines was removed through the spray dryer roof and subsequently passed trough a cyclone that removed the particles. The removed particles were pneumatically transported back to the spray dryer where they were injected into the nozzle cloud.

The Hyaluronate had a molecular weight of 800 kDa and a concentration of about 8 g/liter. The spray dryer had a diameter of about 1.8 m and a height of about 5 m. The drying conditions were adjusted to give a dry powder batch (batch nr. 04PBAC0014) with the desired particle size:

| | |
|---|---|
| Inlet temperature: | 160 °C |
| Outlet temperature: | 68 °C |
| Nozzle atomization pressure: | 3.5 bar |
| Nozzle air temperature: | 50 °C |
| Feed temperature: | 60 °C |

Subsequently, particle size distribution was measured using laser diffraction on a suspension of the powder in isopropanol:

| | |
|---|---|
| $D_{10}$ : | 89 micron |
| $D_{50}$ : | 220 micron |
| $D_{90}$ : | 466 micron |
| Molecular weight: | 796 kDa |
| Hyaluronate concentration: | 493 mg/g |

It is seen, that the molecular weight was nearly unchanged by the drying operation, thus demonstrating that the spray-drying process is very gentle despite the high temperatures applied.

The product fraction between 250 and 500 micron was tested for its dispersibility/solubility, as shown in table 4, and it was found to disperse and dissolve in about 2 minutes, which was much faster than the 15 minutes observed with the non-agglomerated spray dried product. The absorbance of the resulting solution was also measured at 600 nm to assure that no minute particles were remaining in the solution (table 4).

Table **4**

| Atomizer (batch no.) | Solubility Time (min) | Absorbance A$_{600nm}$ |
|---|---|---|
| TFN (04PBAC0014) | 2 | 0.005 |

**Example 7. Molecular weight and skin penetration**

Skin permeation of four different average molecular weight fractions of radiolabeled hyaluronic acid was investigated, using side-by-side diffusion cells with a diameter of 2 cm (effective skin area of 3.14 cm$^2$). The volume of the receptor phase was between 8 and 8.5 ml, and the volume of the donor compartment was between 1 and 1.5 ml.

Dermatomed skin (750 micrometer) from the porcine ear was thawed and placed on one side of the diffusion cell. Any noticeable hairs were carefully clipped with scissors. The other side of the diffusion cell was closed with a silicon disc. The receptor was filled with phosphate-buffered saline (PBS) at pH 7.4. A magnetic stir-bar was introduced.

Three 10 microliter samples of the donor phase (as provided with a cold/hot ratio of 5.64 mg/ml cold to 22.56 microgram/ml hot) were taken to determine the specific activity. The donor phases were then filled with the solution provided: for 5 ml (the volume received), only 3 or 4 cells could be set up.

The cells were placed on a stirrer plate and the experiment performed at ambient temperature. After 5 hours, the entire receptor phase was withdrawn and the radioactivity therein was determined by liquid scintillation counting. The receptor chamber was then refilled with PBS and diffusion was allowed to proceed overnight.

After 22 hours (i.e., 5 hr + 17 hr), the receiver solution was again withdrawn and analyzed for radioactivity. The cells were disassembled and the skin was incubated with 5 ml of SOLUENE® (PerkinElmer) for 48 hours, or until completely dissolved, before adding scintillation fluid and counting for radioactivity. The experimental results are summarized for the four different molecular weight HA fractions in Table 5.

**Table 5.** Summary of results from experiments examining the permeation of radiolabeled HA across excised porcine ear skin *in vitro* after 5 hours (5h) and 22 hours (22h).

|  | HA 1500 KDa (ng/cm2) | HA 800 KDa (ng/cm2) | HA 300 KDa (ng/cm2) | HA 50 KDa (ng/cm2) |
|---|---|---|---|---|
| R1.5h | 14,1 | 38,7 | 168,7 | 524,2 |
| R2.5h | 10,4 | 20,8 | 62,9 | 171,1 |
| R3.5h | 18,8 | 50,3 | 101,0 | |
| R4.5h | | | 63,4 | |
| *Average* | *14,4* | *36,6* | *99,0* | *347,6* |
| *Variation* | *4,2* | *14,9* | *49,8* | *249,7* |
| | | | | |
| *Average Flux in* | *2,9* | *7,3* | *19,8* | *69,5* |
| *(ng/cm2/h)* | 0,8 | 3,0 | 10,0 | 49,9 |
| | | | | |
| R1.22h | 77,4 | 233,2 | 733,3 | 2669,4 |
| R2.22h | 50,9 | 95,6 | 368,1 | 1007,8 |
| R3.22h | 93,4 | 273,6 | 544,1 | |
| R4.22h | | | 360,4 | |
| *Average* | *73,9* | *200,81* | *501,5* | *1838,6* |
| *Variation* | *21,5* | *93,3* | *176,3* | *1174,9* |
| | | | | |
| *Average Flux in* | *3,4* | *9,1* | *22,8* | *83,6* |
| *(ng/cm2/h)* | 1,0 | 4,2 | 8,0 | 53,4 |
| | | | | |
| skin 1 | 14307,3 | | 3806,2 | 1212,2 |
| skin 2 | 14744,5 | | 3337,5 | 1062,9 |
| skin 3 | 18049,1 | | 5780,4 | 1840,9 |
| skin 4 | | | 4055,4 | |
| *Average* | *15700,3* | | *4244,89* | *1372,0* |
| *Variation* | *2045,8* | | *1066,07* | *412,9* |

Typically, 3 or 4 replicates (Table 5: R1, R2, R3, R4) were obtained. Sometimes, however, an experimental problem was evident (e.g., an obvious skin perforation, or a leak from the diffusion cell), in which case only two replicates were possible.

Total recoveries of radioactivity ('mass balance') were less than 100%, primarily because the viscous nature of the donor phases prevented their complete removal from the diffusion cell compartment. The problem increased with increasing HA molecular weight.

These results are visualized in figure 1, and they indicate that there is an apparent dependence of percutaneous transport on HA molecular weight, with a better permeation apparent for the lower molecular weight species. There was no significant difference between the apparent fluxes measured at 5 and 22 hours for any of the HA size fractions studied.

Overall, therefore, HA penetration is dependent on HA molecular weight. The lower the molecular weight the better the skin penetration.

However the amount that penetrates the skin corresponds to a small fraction of the applied radioactivity.

**Example 8. Topical distribution of different molecular weight fractions of HA**

Skin uptake and distribution of two different average molecular weight fractions of flourescently labelled hyaluronic acid was investigated using laser scanning confocal microscopy, the average molecular weights of the HA fractions were 300,000 and 50,000 Da.

The flourescent HA samples were aqueous solutions at a concentration of 1 mg/mL; the flourescent HA solutions were diluted 10-fold with non-labelled HA of identical molecular weight in distilled water before application.

The skin used for HA application was from the porcine ear. Each HA fraction was applied and tested on at least three separate samples of skin originating from different animals. The skin samples were obtained as fresh and were kept stored frozen for no longer than 2 weeks prior to use.

Prior to the experiment, a thawed skin sample (approx. 0.8 cm$^2$) was sandwiched between donor and receptor phases of simple glass diffusion cells and maintained at 32°C. The donor phase was 0.1 ml of labelled HA solution, and the receptor phase was pH 7.4 buffer.

The flourescent HA solutions remained in contact with the skin samples for 3 hours, then excess donor solutions were removed, the skin was washed 3 times with pH 7.4 buffer, and dried gently with tissue.

The skin was immediately mounted on a glass slide, stratum corneum side up, and examined microscopically with no further processing using a LSM 410 Invert Laser Scan Microscope (Carl Zeiss, Germany). The system is equipped with an argon-krypton laser having excitation wavelengths of 568 and 488nm, which were used separately to view the skin and the labelled HA, respectively.

Images were obtained using Plan-neofluar 10x/0.3 and 40x/0.6 objectives, and then subsequently colour-coded digitally so that flourescence from skin was red, and flourescence from the labelled HA was green. The images were color-coded and overlaid (or superimposed) using Zeiss LSM confocal software.

In separate experiments, skin samples were examined in the absence of the flourescent probes. The autoflourescence from porcine skin enabled structural features to be identified. To visualize the distribution of flourescent HA, confocal images were obtained in the xy-plane (i.e., parallel to skin surface). The skin surface (z = 0 $\mu$m) was defined as the imaging plane of brightest flourescence with a morphology characteristic of stratum corneum. Replicate images, using at least three separate pieces of skin, were acquired for each treatment.

Flourescently labelled HA of 50,000 Da (F-HA 0.05) was only sparsely and superficially visible on the surface of the stratum corneum, but the labelled HA fraction was clearly visible around and/or in hair follicles. Figure 2, panel A, shows skin surface (stratum corneum), hair follicle and hair shaft; panel B clearly shows that green flourescence from HA is primarily visible around and/or in the follicle.

The sparse flourescence on the surface of the stratum corneum was confirmed in another skin sample, which did not have a follicle present. Figure 3, panel A, shows a skin surface; panel B shows that only limited flourescence from labelled HA is visible on that surface.

A similar observation was made with the flourescently labelled HA of 300,000 Da (F-HA 0.30), which also was clearly visible around the hair follicles. Figure 4, panel A, shows a skin surface (stratum corneum), hair follicle and hair shaft; panel B shows the green flourescence from labelled HA around the follicle.

However, while the labelled HA fraction of 300,000 Da was also only sparsely and superficially visible on the skin surface, it clearly showed a preferential accumulation between the keratinocytes on the skin surface. Figure 5, panel A, shows a skin surface; panel B shows that while limited flourescence from labelled HA is visible on that surface, it is accumulated between the keratinocytes.

Conclusions are, that low molecular weight HA shows some preferential accumulation on, in and around follicular structures in the skin. This could be one possible permeation pathway for low MW HA products of compositions of the invention, that would be of particular interest with respect to the treatment of hair loss or baldness.

There was no evidence for transport across intact stratum corneum of low MW HA of 0.05 MDa as after the removal of HA, only few fluorescent species were found sparsely and superficially on surface layers. Finally, HA of 0.30 MDa shows preferential accumulation between the keratinocytes on the skin surface. This might contribute to improved skin hydration as the HA barrier could restrict the passive transepidermal water loss from the skin.

**Claims**

1.    A product comprising hyaluronic acid or a salt thereof, which product is dried and agglomerated.

2.    The product according to claim 1, wherein the hyaluronic acid or salt thereof is recombinantly produced, preferably by a Gram-positive bacterium, more preferably by a bacterium of the genus *Bacillus.*

3.    The product according to claim 1 or 2, wherein the hyaluronic acid or salt thereof has a molecular weight in the range of between 300,000 and 3,000,000; preferably in the range of between 400,000 and 2,500,000; more preferably in the range of between 500,000 and 2,000,000; and most preferably in the range of between 600,000 and 1,800,000.

4. The product according to claim 1 or 2, wherein the hyaluronic acid or salt thereof has a low average molecular weight in the range of between 10,000 and 800,000 Da; preferably in the range of between 20,000 and 600,000 Da; more preferably in the range of between 30,000 and 500,000 Da; even more preferably in the range of between 40,000 and 400,000 Da; and most preferably in the range of between 50,000 and 300,000 Da.

5. The product according to any of claims 1 - 4, which comprises an inorganic salt of hyaluronic acid, preferably sodium hyaluronate, potassium hyaluronate, ammonium hyaluronate, calcium hyaluronate, magnesium hyaluronate, zinc hyaluronate, or cobalt hyaluronate.

6. The product according to any of claims 1 - 5, which comprises crosslinked hyaluronic acid or salt thereof, preferably the hyaluronic acid is crosslinked with boric acid, and more preferably the crosslinked hyaluronic acid comprises borate esters.

7. The product according to any of claims 1 - 6, which comprises less than 5% moisture, preferably less than 2%, and most preferably less than 1% moisture, as determined herein.

8. The product according to any of claims 1 - 7, which has a particle size the 50 percentile of which, $D_{50}$, is between 10 and 1,000 microns, preferably between 100 and 1,000 microns, more preferably between 150 and 900 microns, and even more preferably between 200 and 800 microns, as determined by laser diffraction measurement of the particles suspended in isopropanol.

9. The product according to any of claims 1 - 8, which has a dispersibility of less than 30 minutes in an aqueous solvent, preferably less than 20 minutes, more preferably less than 15 minutes, more preferably less than 10 minutes, more preferably less than 5 minutes, more preferably less than 3 minutes, more preferably less than 2 minutes, and most preferably less than 1 minute, as determined herein.

10. The product according to any of claims 1 - 9, which has a solubility of less than 60 minutes in an aqueous solvent, preferably less than 45 minutes, more preferably less than 40 minutes, even more preferably less than 35 minutes, 30 minutes, 25 minutes, 20 minutes, 15 minutes, 10 minutes, and most preferably less than 5 minutes, as determined herein.

11. The product according to any of claims 1 - 10, which also comprises an active ingredient.

12. The product according to claim 11, wherein the active ingredient is a pharmacologically active substance.

13. The product according to any of claims 1 - 12, which also comprises a water-soluble excipient, preferably lactose.

14. A composition comprising a product as defined in any of claims 1 - 13 and an active ingredient, preferably the active ingredient is a pharmacologically active agent.

15. The composition according to claim 14, which also comprises a water-soluble excipient, preferably lactose.

16. A pharmaceutical composition comprising an effective amount of a product as defined in any of claims 1 - 13, together with a pharmaceutically acceptable carrier, excipient or diluent.

17. A pharmaceutical composition comprising an effective amount of a product as defined in any of claims 1 - 13 as a vehicle, together with a pharmacologically active agent.

18. A cosmetic article comprising as an active ingredient an effective amount of a product as defined in any of claims 1 - 13 or a composition as defined in any of claims 14 - 17.

19. A sanitary, medical or surgical article comprising a product as defined in any of claims 1 - 13 or a composition as defined in any of claims 14 - 17; preferably the article is a diaper, a sanitary towel, a surgical sponge, a wound healing sponge, or a part comprised in a band aid or other wound dressing material.

20. A medicament capsule or microcapsule comprising a product as defined in any of claims 1 - 13 or a composition as defined in any of claims 14 - 17.

21. A method of producing a dried and agglomerated product comprising hyaluronic acid or a salt thereof, the method comprising the steps of:

   a) drying a product comprising hyaluronic acid or a salt thereof; and
   b) agglomerating the dried product,

   wherein the agglomerating step is done using a fluidised bed or a water-solvent mixture.

22. The method according to claim 21, wherein the hyaluronic acid or salt thereof is recombinantly produced, preferably by a Gram-positive bacterium, more preferably by a bacterium of the genus *Bacillus.*

23. The method according to claim 21 or 22, wherein the hyaluronic acid or salt thereof has a molecular weight in the range of between 300,000 and 3,000,000; preferably in the range of between 400,000 and 2,500,000; more preferably in the range of between 500,000 and 2,000,000; and most preferably in the range of between 600,000 and 1,800,000.

24. The method according to claim 21 or 22, wherein the hyaluronic acid or salt thereof has a low average molecular weight in the range of between 10,000 and 800,000 Da; preferably in the range of between 20,000 and 600,000 Da; more preferably in the range of between 30,000 and 500,000 Da; even more preferably in the range of between 40,000 and 400,000 Da; and most preferably in the range of between 50,000 and 300,000 Da.

25. The method according to any of claims 21 - 24, wherein the product comprises an inorganic salt of hyaluronic acid, preferably sodium hyaluronate, potassium hyaluronate, ammonium hyaluronate, calcium hyaluronate, magnesium hyaluronate, zinc hyaluronate, or cobalt hyaluronate.

26. The method according to any of claims 21 - 25, wherein the product comprises crosslinked hyaluronic acid or salt thereof, preferably the hyaluronic acid is crosslinked with boric acid, and more preferably the crosslinked hyaluronic acid comprises borate esters.

27. The method according to any of claims 21 - 26, wherein the drying step is done using a spray-dryer or a Rotary Atomizer, preferably a Two-Fluid-Nozzle (TFN) spray-dryer.

28. The method according to claim 27, wherein the spray-drying was done using a TFN spray-dryer and the following condition ranges:

| | |
|---|---|
| Inlet temperature: | 100 - 200 °C |
| Outlet temperature: | 40 - 90 °C |
| Nozzle atomization pressure: | 2 - 10 bar |
| Nozzle air temperature: | 40 - 100 °C |
| Feed temperature: | 40 - 100 °C |

29. The method according to claim 21, wherein the fluidised bed inlet temperature is in the range of 30 - 80 °C, and wherein the aqueous solution of the powder is sprayed on the dried powder at a rate corresponding to an outlet temperature of 25 - 50 °C.

30. The method according to claim 21, wherein the agglomerating step is done using a water-solvent mixture having a water-solvent ratio of between 0:100 and 50:50, and more preferably having a water-solvent ratio of between 0:100 and 20:80.

31. The method according to claim 30, wherein the solvent is an alcohol, preferably iso-propanol.

32. The method according to any of claims 21 - 29, wherein the drying and agglomerating is done in one step, preferably using a fluidised spray-dryer.

**33.** Use of a product as defined in any of claims 1-13 or a composition as defined in any of claims 14 -17 for the manufacture of a medicament for the treatment of osteoarthritis.

**34.** Use of a product as defined in any of claims 1-13 or a composition as defined in any of claims 14 - 17 for the manufacture of a medicament for an ophtalmological treatment.

**35.** Use of a product as defined in any of claims 1-13 or a composition as defined in any of claims 14 - 17 for the manufacture of a medicament for the treatment of cancer.

**36.** Use of a product as defined in any of claims 1-13 or a composition as defined in any of claims 14 - 17 for the manufacture of a medicament for the treatment of a wound.

**37.** Use of a product as defined in any of claims 1-13 or a composition as defined in any of claims 14 - 17 for the manufacture of a medicament for angiogenesis.

**38.** Use of a product as defined in any of claims 1-13 or a composition as defined in any of claims 14 - 17 for the manufacture of a moisturizer.

**39.** Use of a product as defined in any of claims 1-13 or a composition as defined in any of claims 14 - 17 for the manufacture of a medicament for the treatment of hair loss or baldness.


**Patentansprüche**

**1.** Produkt, umfassend Hyaluronsäure oder ein Salz davon, wobei das Produkt getrocknet und agglomeriert ist.

**2.** Produkt nach Anspruch 1, wobei die Hyaluronsäure oder das Salz davon rekombinant hergestellt ist, vorzugsweise durch ein gram-positives Bakterium, stärker bevorzugt durch ein Bakterium der Gattung *Bacillus.*

**3.** Produkt nach Anspruch 1 oder 2, wobei die Hyaluronsäure oder das Salz davon ein Molekulargewicht in dem Bereich von zwischen 300.000 und 3.000.000 aufweist; vorzugsweise in dem Bereich von zwischen 400.000 und 2.500.000; stärker bevorzugt in dem Bereich von zwischen 500.000 und 2.000.000; und am stärksten bevorzugt in dem Bereich von zwischen 600.000 und 1.800.000.

**4.** Produkt nach Anspruch 1 oder 2, wobei die Hyaluronsäure oder das Salz davon ein niedriges durchschnittliches Molekulargewicht in dem Bereich von zwischen 10.000 und 800.000 Da aufweist; vorzugsweise in dem Bereich von zwischen 20.000 und 600.000 Da; stärker bevorzugt in dem Bereich von zwischen 30.000 und 500.000 Da; noch stärker bevorzugt in dem Bereich von zwischen 40.000 und 400.000 Da; und am stärksten bevorzugt in dem Bereich von zwischen 50.000 und 300.000 Da.

**5.** Produkt nach einem beliebigen der Ansprüche 1 - 4, das ein anorganisches Salz der Hyaluronsäure umfasst, vorzugsweise Natriumhyaluronat, Kaliumhyaluronat, Ammoniumhyaluronat, Calciumhyaluronat, Magnesiumhyaluronat, Zinkhyaluronat oder Kobalthyaluronat.

**6.** Produkt nach einem beliebigen der Ansprüche 1 - 5, das vernetzte Hyaluronsäure oder ein Salz davon umfasst, vorzugsweise ist die Hyaluronsäure mit Borsäure vernetzt und stärker bevorzugt umfasst die vernetzte Hyaluronsäure Boratester.

**7.** Produkt nach einem beliebigen der Ansprüche 1 - 6, das weniger als 5% Feuchtigkeit umfasst, vorzugsweise weniger als 2% und am stärksten bevorzugt weniger als 1% Feuchtigkeit, wie hierin bestimmt.

**8.** Produkt nach einem beliebigen der Ansprüche 1 - 7, das eine Partikelgröße der 50. Percentile, $D_{50}$, aufweist, die zwischen 10 und 1000 Mikrometer beträgt, vorzugsweise zwischen 100 und 1000 Mikrometer, stärker bevorzugt zwischen 150 und 900 Mikrometer, und noch stärker bevorzugt zwischen 200 und 800 Mikrometer, wie durch Laserdiffraktionsmessung der in Isopropanol suspendierten Partikel bestimmt.

**9.** Produkt nach einem beliebigen der Ansprüche 1 - 8, das eine Dispergierbarkeit von weniger als 30 Minuten in einem wässrigen Lösungsmittel aufweist, vorzugsweise weniger als 20 Minuten, stärker bevorzugt weniger als 15 Minuten, stärker bevorzugt weniger als 10 Minuten, stärker bevorzugt weniger als 5 Minuten, stärker bevorzugt weniger als 3 Minuten, stärker bevorzugt weniger als 2 Minuten und am stärksten bevorzugt weniger als 1 Minute, wie hierin bestimmt.

10. Produkt nach einem beliebigen der Ansprüche 1 - 9, das eine Löslichkeit von weniger als 60 Minuten in einem wässrigen Lösungsmittel aufweist, vorzugsweise weniger als 45 Minuten, stärker bevorzugt weniger als 40 Minuten, noch stärker bevorzugt weniger als 35 Minuten, 30 Minuten, 25 Minuten, 20 Minuten, 15 Minuten, 10 Minuten und am stärksten bevorzugt weniger als 5 Minuten, wie hierin bestimmt.

11. Produkt nach einem beliebigen der Ansprüche 1 - 10, das auch einen aktiven Bestandteil umfasst.

12. Produkt nach Anspruch 11, wobei der aktive Bestandteil eine pharmakologisch aktive Substanz ist.

13. Produkt nach einem beliebigen der Ansprüche 1 - 12, das auch einen wasserlöslichen Hilfsstoff umfasst, vorzugsweise Laktose.

14. Zusammensetzung, umfassend ein wie in einem beliebigen der Ansprüche 1 - 13 definiertes Produkt und einen aktiven Bestandteil, vorzugsweise ist der aktive Bestandteil ein pharmakologisch aktiver Wirkstoff.

15. Zusammensetzung nach Anspruch 14, die ebenfalls einen wasserlöslichen Hilfsstoff umfasst, vorzugsweise Lactose.

16. Pharmazeutische Zusammensetzung, umfassend eine wirksame Menge eines wie in einem beliebigen der Ansprüche 1 - 13 definierten Produkts, zusammen mit einem pharmazeutisch verträglichen Träger, Hilfsstoff oder Verdünnungsmittel.

17. Pharmazeutische Zusammensetzung, umfassend eine wirksame Menge eines wie in einem beliebigen der Ansprüche 1 - 13 definierten Produkts als Vehikel, zusammen mit einem pharmakologisch aktiven Wirkstoff.

18. Kosmetikartikel, umfassend als aktiven Bestandteil eine wirksame Menge eines wie in einem beliebigen der Ansprüche 1 - 13 definierten Produkts oder einer wie in einem beliebigen der Ansprüche 14 - 17 definierten Zusammensetzung.

19. Hygiene-, Medizin- oder Chirurgieartikel, umfassend ein wie in einem beliebigen der Ansprüche 1 - 13 definiertes Produkt oder eine wie in einem beliebigen der Ansprüche 14 - 17 definierte Zusammensetzung; vorzugsweise ist der Artikel eine Windel, eine Binde, ein chirurgischer Schwamm, ein Wundheilungsschwamm oder ein Teil, der in einem Pflaster oder einem anderen Wundauflagematerial umfasst ist.

20. Medikamentenkapsel oder Mikrokapsel, umfassend ein wie in einem beliebigen der Ansprüche 1 - 13 definiertes Produkt oder eine wie in einem beliebigen der Ansprüche 14 - 17 definierte Zusammensetzung.

21. Verfahren zum Herstellen eines getrockneten und agglomerierten Produkts, das Hyaluronsäure oder ein Salz davon umfasst, wobei das Verfahren die Schritte umfasst:

a) Trocknen eines Produkts, das Hyaluronsäure oder ein Salz davon umfasst; und
b) Agglomerieren des getrockneten Produkts,

wobei der Agglomerierungsschritt unter Verwendung einer Wirbelschicht oder eines Wasser-Lösungsmittel-Gemischs durchgeführt wird.

22. Verfahren nach Anspruch 21, wobei die Hyaluronsäure oder das Salz davon rekombinant hergestellt wird, vorzugsweise durch ein gram-positives Bakterium, stärker bevorzugt durch ein Bakterium der Gattung *Bacillus.*

23. Verfahren nach Anspruch 21 oder 22, wobei die Hyaluronsäure oder das Salz davon ein Molekulargewicht in dem Bereich von zwischen 300.000 und 3.000.000 aufweist; vorzugsweise in dem Bereich von zwischen 400.000 und 2.500.000; stärker bevorzugt in dem Bereich von zwischen 500.000 und 2.000.000; und am stärksten bevorzugt in dem Bereich von zwischen 600.000 und 1.800.000.

24. Verfahren nach Anspruch 21 oder 22, wobei die Hyaluronsäure oder das Salz davon ein niedriges durchschnittliches Molekulargewicht in dem Bereich von zwischen 10.000 und 800.000 Da aufweist; vorzugsweise in dem Bereich von zwischen 20.000 und 600.000 Da; stärker bevorzugt in dem Bereich von zwischen 30.000 und 500.000 Da; noch stärker bevorzugt in dem Bereich von zwischen 40.000 und 400.000 Da; und am stärksten bevorzugt in dem Bereich von zwischen 50.000 und 300.000 Da.

25. Verfahren nach einem beliebigen der Ansprüche 21 - 24, wobei das Produkt ein anorganisches Salz der Hyaluronsäure umfasst, vorzugsweise Natriumhyaluronat, Kaliumhyaluronat, Ammoniumhyaluronat, Calciumhyaluronat, Magnesiumhyaluronat, Zinkhyaluronat oder Kobalthyaluronat.

26. Verfahren nach einem beliebigen der Ansprüche 21 - 25, wobei das Produkt vernetzte Hyaluronsäure oder ein Salz davon umfasst, vorzugsweise ist die Hyaluronsäure mit Borsäure vernetzt und stärker bevorzugt umfasst die vernetzte Hyaluronsäure Boratester.

27. Verfahren nach einem beliebigen der Ansprüche 21 - 26, wobei der Trocknungsschritt unter Verwendung eines Sprühtrockners oder eines Rotationszerstäubers durchgeführt wird, vorzugsweise eines Zwei-Stoff-Düse- (TFN) -Sprühtrockners.

28. Verfahren nach Anspruch 27, wobei das Sprühtrocknen durchgeführt wurde unter Verwendung eines TFN-Sprühtrockners und den folgenden Bedingungsbereichen:

| | |
|---|---|
| Eintrittstemperatur: | 100 - 200 °C |
| Austrittstemperatur: | 40 - 90 °C |
| Düsenzerstäubungsdruck: | 2 - 10 bar |
| Düsenlufttemperatur: | 40 - 100 °C |
| Vorlauftemperatur: | 40 - 100 °C |

29. Verfahren nach Anspruch 21, wobei die Wirbelschicht-Eintrittstemperatur in dem Bereich von 30 - 80 °C liegt, und wobei die wässrige Lösung des Pulvers auf das getrocknete Pulver in einer Geschwindigkeit aufgesprüht wird, die einer Austrittstemperatur von 25 - 50 °C entspricht.

30. Verfahren nach Anspruch 21, wobei der Agglomerierungsschritt durchgeführt wird unter Verwendung eines Wasser-Lösungsmittel-Gemischs mit einem Wasser-Lösungsmittel-Verhältnis von zwischen 0:100 und 50:50, und stärker bevorzugt mit einem Wasser-Lösungsmittel-Verhältnis von zwischen 0:100 und 20:80.

31. Verfahren nach Anspruch 30, wobei das Lösungsmittel ein Alkohol ist, vorzugsweise Isopropanol.

32. Verfahren nach einem beliebigen der Ansprüche 21 - 29, wobei das Trocknen und Agglomerieren in einem Schritt durchgeführt wird, vorzugsweise unter Verwendung eines Wirbelschicht-Sprühtrockners.

33. Verwendung eines wie in einem beliebigen der Ansprüche 1 - 13 definierten Produkts oder einer wie in einem beliebigen der Ansprüche 14 - 17 definierten Zusammensetzung für die Herstellung eines Medikaments zur Behandlung von Osteoarthritis.

34. Verwendung eines wie in einem beliebigen der Ansprüche 1 - 13 definierten Produkts oder einer wie in einem beliebigen der Ansprüche 14 - 17 definierten Zusammensetzung für die Herstellung eines Medikaments für eine ophthalmologische Behandlung.

35. Verwendung eines wie in einem beliebigen der Ansprüche 1 - 13 definierten Produkts oder einer wie in einem beliebigen der Ansprüche 14 - 17 definierten Zusammensetzung für die Herstellung eines Medikaments für die Behandlung von Krebs.

36. Verwendung eines wie in einem beliebigen der Ansprüche 1 - 13 definierten Produkts oder einer wie in einem beliebigen der Ansprüche 14 - 17 definierten Zusammensetzung für die Herstellung eines Medikaments für die Behandlung einer Wunde.

37. Verwendung eines wie in einem beliebigen der Ansprüche 1 - 13 definierten Produkts oder einer wie in einem beliebigen der Ansprüche 14 - 17 definierten Zusammensetzung für die Herstellung eines Medikaments für Angiogenese.

38. Verwendung eines wie in einem beliebigen der Ansprüche 1 - 13 definierten Produkts oder einer wie in einem beliebigen der Ansprüche 14 - 17 definierten Zusammensetzung für die Herstellung eines Befeuchtungsmittels.

39. Verwendung eines wie in einem beliebigen der Ansprüche 1 - 13 definierten Produkts oder einer wie in einem beliebigen der Ansprüche 14 - 17 definierten Zusammensetzung für die Herstellung eines Medikaments für die Behandlung von Haarverlust oder Glatzköpfigkeit.


**Revendications**

1. Produit comprenant de l'acide hyaluronique ou un sel de celui-ci, ledit produit étant séché et aggloméré.

2. Produit selon la revendication 1, dans lequel l'acide hyaluronique ou sel de celui-ci est produit par recombinaison, de préférence par une bactérie Gram-positive, plus préférablement par une bactérie du genre *Bacillus*.

3. Produit selon les revendications 1 ou 2, dans lequel l'acide hyaluronique ou sel celui-ci a un poids moléculaire dans la plage comprise entre 300 000 et 3 000 000 ; de préférence dans la plage comprise entre 400 000 et 2 500 000 ; plus préférablement dans la plage comprise entre 500 000 et 2 000 000 ; et de manière encore plus préférée dans la plage comprise entre 600 000 et 1 800 000.

4. Produit selon les revendications 1 ou 2, dans lequel l'acide hyaluronique ou sel de celui-ci a un bas poids moléculaire moyen dans la plage comprise entre 10 000 et 800 000 Da ; de préférence dans la plage comprise entre 20 000 et 600 000 Da ; plus préférablement dans la plage comprise entre 30 000 et 500 000 Da ; plus préférablement encore dans la plage comprise entre 40 000 et 400 000 Da ; et de manière encore plus préférée dans la plage comprise entre 50 000 et 300 000 Da.

5. Produit selon l'une quelconque des revendications 1 à 4, qui comprend un sel inorganique d'acide hyaluronique, de préférence un hyaluronate de sodium, un hyaluronate de potassium, un hyaluronate d'ammonium, un hyaluronate de calcium, un hyaluronate de magnésium, un hyaluronate de zinc, ou un hyaluronate de cobalt.

6. Produit selon l'une quelconque des revendications 1 à 5, qui comprend un acide hyaluronique réticulé ou un sel de celui-ci, l'acide hyaluronique étant de préférence réticulé avec de l'acide borique, et plus préférablement l'acide hyaluronique réticulé comprenant des esters de borate.

7. Produit selon l'une quelconque des revendications 1 à 6, qui comprend moins de 5 % d'humidité, de préférence moins de 2 %, et de manière encore plus préférée moins de 1 % d'humidité, comme déterminé dans la présente.

8. Produit selon l'une quelconque des revendications 1 à 7, qui a une taille de particule dont le centile 50, $D_{50}$, est compris entre 10 et 1000 microns, de préférence entre 100 et 1000 microns, plus préférablement entre 150 et 900 microns, et plus préférablement encore entre 200 et 800 microns, comme déterminé par la mesure de la diffraction laser des particules en suspension dans fisopropanol.

9. Produit selon l'une quelconque des revendications 1 à 8, qui a une dispersabilité inférieure à 30 minutes dans un solvant aqueux, de préférence inférieure à 20 minutes, plus préférablement inférieure à 15 minutes, plus préférablement inférieure à 10 minutes, plus préférablement inférieure à 5 minutes, plus préférablement inférieure à 3 minutes, plus préférablement inférieure à 2 minutes, et de manière encore plus préférée inférieure à 1 minute, comme déterminé dans la présente.

10. Produit selon l'une quelconque des revendications 1 à 9, qui a une solubilité inférieure à 60 minutes dans un solvant aqueux, de préférence inférieure à 45 minutes, plus préférablement inférieure à 40 minutes, plus préférablement encore inférieure à 35 minutes, 30 minutes, 25 minutes, 20 minutes, 15 minutes, 10 minutes, et de manière encore plus préférée inférieure à 5 minutes, comme déterminé dans la présente.

11. Produit selon l'une quelconque des revendications 1 à 10, qui comprend également un principe actif.

**12.** Produit selon la revendication 11, dans lequel le principe actif est une substance pharmacologiquement active.

**13.** Produit selon l'une quelconque des revendications 1 à 12, qui comprend également un excipient hydrosoluble, de préférence le lactose.

**14.** Composition comprenant un produit selon l'une quelconque des revendications 1 à 13 et un principe actif, le principe actif étant de préférence un agent pharmacologiquement actif.

**15.** Composition selon la revendication 14, qui comprend également un excipient hydrosoluble, de préférence le lactose.

**16.** Composition pharmaceutique comprenant une quantité efficace d'un produit selon l'une quelconque des revendications 1 à 13, avec un véhicule, un excipient ou un diluant pharmaceutiquement acceptable.

**17.** Composition pharmaceutique comprenant une quantité efficace d'un produit selon l'une quelconque des revendications 1 à 13 à titre de véhicule, avec un agent pharmacologiquement actif.

**18.** Article cosmétique comprenant à titre de principe actif une quantité efficace d'un produit selon l'une quelconque des revendications 1 à 13 ou une composition selon l'une quelconque des revendications 14 à 17.

**19.** Article hygiénique, médical ou chirurgical comprenant un produit selon l'une quelconque des revendications 1 à 13 ou une composition selon l'une quelconque des revendications 14 à 17 ; l'article étant de préférence une couche, une serviette hygiénique, une éponge chirurgicale, une éponge pour la cicatrisation des plaies, ou constituant une partie contenue dans un pansement adhésif ou autre matériau de pansement pour plaies.

**20.** Capsule ou microcapsule de médicament comprenant un produit selon l'une quelconque des revendications 1 à 13 ou une composition selon l'une quelconque des revendications 14 à 17.

**21.** Méthode de production d'un produit séché et aggloméré comprenant de l'acide hyaluronique ou un sel de celui-ci, la méthode comprenant les étapes suivantes :

   a) séchage d'un produit comprenant de l'acide hyaluronique ou un sel de celui-ci ; et
   b) agglomération du produit séché,

où l'étape d'agglomération est réalisée en utilisant un lit fluidisé ou un mélange eau-solvant.

**22.** Méthode selon la revendication 21, dans laquelle l'acide hyaluronique ou un sel de celui-ci est produit par recombinaison, de préférence par une bactérie Gram-positive, plus préférablement par une bactérie du genre *Bacillus.*

**23.** Méthode selon la revendication 21 ou 22, dans laquelle l'acide hyaluronique ou un sel de celui-ci a un poids moléculaire dans la plage comprise entre 300 000 et 3 000 000 ; de préférence dans la plage comprise entre 400 000 et 2 500 000 ; plus préférablement dans la plage comprise entre 500 000 et 2 000 000 ; et de manière encore plus préférée dans la plage comprise entre 600 000 et 1 800 000.

**24.** Méthode selon la revendication 21 ou 22, dans laquelle l'acide hyaluronique ou un sel de celui-ci a un bas poids moléculaire moyen dans la plage comprise entre 10 000 et 800 000 Da ; de préférence dans la plage comprise entre 20 000 et 600 000 Da ; plus préférablement dans la plage comprise entre 30 000 et 500 000 Da ; plus préférablement encore dans la plage comprise entre 40 000 et 400 000 Da ; et de manière encore plus préférée dans la plage comprise entre 50 000 et 300 000 Da.

**25.** Méthode selon l'une quelconque des revendications 21 à 24, dans laquelle le produit comprend un sel inorganique d'acide hyaluronique, de préférence un hyaluronate de sodium, un hyaluronate de potassium, un hyaluronate d'ammonium, un hyaluronate de calcium, un hyaluronate de magnésium, un hyaluronate de zinc, ou un hyaluronate de cobalt.

**26.** Méthode selon l'une quelconque des revendications 21 à 25, dans laquelle le produit comprend un acide hyaluronique réticulé ou un sel de celui-ci, l'acide hyaluronique étant de préférence réticulé avec de l'acide borique, et plus préférablement, l'acide hyaluronique réticulé comprenant des esters de borate.

**27.** Méthode selon l'une quelconque des revendications 21 à 26, dans laquelle l'étape de séchage est mise en oeuvre à l'aide d'un sécheur par atomisation ou un atomiseur rotatif, de préférence un sécheur par atomisation à buse à double fluide (TFN).

**28.** Méthode selon la revendication 27, dans laquelle le séchage par atomisation a été mis en oeuvre à l'aide d'un sécheur par atomisation TFN et dans les plages de conditions suivantes :

| | |
|---|---|
| Température d'entrée : | 100-200 °C |
| Température de sortie : | 40-90 °C |
| Pression d'atomisation de buse : | 2 à 10 bar |
| Température d'air de buse : | 40-100 °C |
| Température d'alimentation : | 40-100 °C |

**29.** Méthode selon la revendication 21, dans laquelle la température d'entrée du lit fluidisé est dans la plage de 30 à 80 °C, et dans laquelle la solution aqueuse de la poudre est pulvérisée sur la poudre séchée à un taux correspondant à une température de sortie de 25 à 50 °C.

**30.** Méthode selon la revendication 21, dans laquelle l'étape d'agglomération est mise en oeuvre à l'aide d'un mélange eau-solvant ayant un rapport eau-solvant compris entre 0:100 et 50:50, et de préférence ayant un rapport eau-solvant compris entre 0:100 et 20:80.

**31.** Méthode selon la revendication 30, dans laquelle le solvant est un alcool, de préférence fisopropanol.

**32.** Méthode selon l'une quelconque des revendications 21 à 29, dans laquelle le séchage et l'agglomération sont mis en oeuvre en une étape, de préférence à l'aide d'un sécheur par atomisation fluidisé.

**33.** Utilisation d'un produit selon l'une quelconque des revendications 1 à 13 ou d'une composition selon l'une quelconque des revendications 14 à 17 dans la fabrication d'un médicament destiné à traiter l'arthrose.

**34.** Utilisation d'un produit selon l'une quelconque des revendications 1 à 13 ou d'une composition selon l'une quelconque des revendications 14 à 17 dans la fabrication d'un médicament pour un traitement ophtalmologique.

**35.** Utilisation d'un produit selon l'une quelconque des revendications 1 à 13 ou d'une composition selon l'une quelconque des revendications 14 à 17 dans la fabrication d'un médicament destiné à traiter le cancer.

**36.** Utilisation d'un produit selon l'une quelconque des revendications 1 à 13 ou d'une composition selon l'une quelconque des revendications 14 à 17 dans la fabrication d'un médicament destiné à traiter une plaie.

**37.** Utilisation d'un produit selon l'une quelconque des revendications 1 à 13 ou d'une composition selon l'une quelconque des revendications 14 à 17 dans la fabrication d'un médicament pour angiogenèse.

**38.** Utilisation d'un produit selon l'une quelconque des revendications 1 à 13 ou d'une composition selon l'une quelconque des revendications 14 à 17 dans la fabrication d'un hydratant.

**39.** Utilisation d'un produit selon l'une quelconque des revendications 1 à 13 ou d'une composition selon l'une quelconque des revendications 14 à 17 dans la fabrication d'un médicament destiné à traiter la chute des cheveux ou la calvitie.

## Figure 1

**Fig. 2 - Panel A**

**Fig. 2 - Panel B**

Fig. 3 - Panel A

Fig. 3 - Panel B

**Fig. 4 - Panel A**

**Fig. 4 - Panel B**

**Fig. 5 - Panel A**

**Fig. 5 - Panel B**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- GB 2218429 A
- US 4754027 A
- WO 0209787 A
- US 6255076 B
- US 5955310 A
- US 5955310 B
- WO 9425612 A
- US 4801539 A
- EP 0694616 A
- WO 03054163 A
- WO 9923227 A

- WO 9951265 A
- WO 0027437 A
- WO 9822598 A
- US 5891701 A
- US 6020484 A
- EP 0161887 B1
- US 4957744 A
- US 5616568 A
- US 5652347 A
- US 5874417 A

**Non-patent literature cited in the description**

- **LAURENT T. C. ; FRASER J. R. E.** *FASEB J.,* 1992, vol. 6, 2397-2404
- Proteoglycans and hyaluronan in morphogenesis and differentiation. **TOOLE B.P.** Cell Biology of the Extracellular Matrix. Plenum, 1991, 305-341
- **INNIS et al.** PCR Protocols: A Guide to Methods and Application. Academic Press, 1990
- **KUNST et al.** The complete genome sequence of the Gram-positive bacterium Bacillus subtilis. *Nature,* 20 November 1997, vol. 390, 249-256
- **VIIIA-KAMAROFF et al.** *Proceedings of the National Academy of Sciences USA,* 1978, vol. 75, 3727-3731
- **DEBOER et al.** *Proceedings of the National Academy of Sciences USA,* 1983, vol. 80, 21-25
- Useful proteins from recombinant bacteria. *Scientific American,* 1980, vol. 242, 74-94
- **SAMBROOK ; FRITSCH ; MANIATUS.** Molecular Cloning, A Laboratory Manual. Cold Spring Harbor, 1989
- **VOSKUIL et al.** *Molecular Microbiology,* 1995, vol. 17, 271-279
- **AGAISSE ; LERECLUS.** *Molecular Microbiology,* 1994, vol. 13, 97-107
- **HUE et al.** *Journal of Bacteriology,* 1995, vol. 177, 3465-3471
- **SIMONEN ; PALVA.** *Microbiological Reviews,* 1993, vol. 57, 109-137

- Protein Purification. VCH Publishers, 1989
- **DEANGELIS, P. L.** *Cell. Mol. Life Sci.,* 1999, vol. 56, 670-682
- *Proc. Natl. Acad. Sci. USA.,* 2001, vol. 98, 4658-4663
- **CHANG ; COHEN.** *Molecular General Genetics,* 1979, vol. 168, 111-115
- **YOUNG ; SPIZIZEN.** *Journal of Bacteriology,* 1961, vol. 81, 823-829
- **DUBNAU ; DAVIDOFF-ABELSON.** *Journal of Molecular Biology,* 1971, vol. 56, 209-221
- **SHIGEKAWA ; DOWER.** *Biotechniques,* 1988, vol. 6, 742-751
- **KOEHLER ; THORNE.** *Journal of Bacteriology,* 1987, vol. 169, 5271-5278
- **BITTER ; MUIR.** *Anal Biochem.,* 1962, vol. 4, 330-334
- **UENO et al.** *Chem. Pharm. Bull.,* 1988, vol. 36, 4971-4975
- **WYATT.** *Anal. Chim. Acta,* 1993, vol. 272, 1-40
- Light Scattering University DAWN Course Manual. DAWN EOS Manual. Wyatt Technology Corporation, 1999
- **T. MIYAZAKI et al.** *Polymer Degradation and Stability,* 2001, vol. 74, 77-85